(19) Europäisches Patentamt
European Patent Office
Office européen des brevets

(11) EP 3 067 428 A1

(12) EUROPEAN PATENT APPLICATION

(43) Date of publication:
14.09.2016 Bulletin 2016/37

(51) Int Cl.:
C12P 19/14 (2006.01)
C08L 97/02 (2006.01)
C13K 13/00 (2006.01)
C08H 8/00 (2010.01)
C13K 1/00 (2006.01)

(21) Application number: 15425019.5

(22) Date of filing: 12.03.2015

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(71) Applicants:
• BETA RENEWABLES S.p.A.
15057 Tortona (AL) (IT)
• Novozymes A/S
2880 Bagsværd (DK)

(72) Inventors:
• Riva, Daniele
I-18100 Imperia (IT)
• Purrotti, Micol
I-10050 Borgone Susa (TO) (IT)

• Montecchi, Donatello
I-50026 San Casciano in Val di Pesa (FI) (IT)
• Crippa, Tommaso
I-28100 Novara (IT)
• Ottonello, Piero
I-20143 Milano (IT)
• Prefumo, Chiara
I-16163 GENOVA (IT)
• Ferrero, Simone
I-15057 Tortona (AL) (IT)
• Paravisi, Stefano
I-15057 Tortona (AL) (IT)
• Belfrage, Johan
Cupar KY15 4QJ (GB)
• Frickmann, Jesper
Raleigh, North Carolina 27614 (US)

(74) Representative: Zambardino, Umberto
Botti & Ferrari S.r.l.
Via Cappellini, 11
20124 Milano (IT)

(54) **A PROCESS FOR PRODUCING A HYDROLYZED MIXTURE FROM A PRE-TREATED LIGNO-CELLULOSIC SLURRY COMPRISING A SLURRY LIQUID AND SLURRY SOLIDS**

(57) It is disclosed a process for producing a hydrolyzed mixture from a pre-treated ligno-cellulosic slurry comprising a slurry liquid and slurry solids. In the disclosed process, at least a portion of a cellulase from an enzyme cocktail having an initial cellulase activity is adsorbed onto the slurry solids of the pre-treated ligno-cellulosic biomass slurry to create an adsorbed cellulase, wherein the adsorbed cellulase has an adsorbed cellulase activity which is greater than 45% of the initial cellulase activity. The pre-treated ligno-cellulosic slurry with adsorbed enzymes is subjected to enzymatic hydrolysis to produce the hydrolyzed mixture. The enzymatic hydrolysis may comprise more than one hydrolysis step, which may be continuous or batch.

Figure 1

**Description**

BACKGROUND

The ligno-cellulosic feedstocks may represent an alternative to fossil oil as a source of biofuels and other sustainable bio-based products, provided that cost-competitive conversion process are developed.

Different pre-treatments have been developed so far for increasing the accessibility of biological agents to the cellulose and hemicellulose in the ligno-cellulosic feedstock. Namely, biological agents such as enzymes are used to hydrolyze glucans and xylans contained in the ligno-cellulosic feedstock to produce a fermentable hydrolyzate comprising monomeric sugars. The enzymatic hydrolysis of cellulose and hemicellulose is a multi-step reaction that takes place in a heterogeneous system; for instance, insoluble cellulose is initially broken down at the solid-liquid interface via the synergistic action of endoglucanases and exo-glucanases/ cellobiohydrolases. This initial degradation is accompanied by further liquid phase hydrolysis of soluble intermediate products, for instance short cellulooligosaccharides and cellobiose, that are catalytically cleaved to produce glucose by the action of β-glucosidase.

Intense research efforts are currently directed towards improving the hydrolytic degradation of ligno-cellulosic biomass. These efforts include improvements of both the biomass pre-treatment technologies and the cellulolytic enzymes that catalyze the conversion of the ligno-cellulose to glucose and pentoses. Significant progress has already been made with respect to selecting and developing better enzymes with improved stability, higher specificity, and faster action on solid lignocellulose substrates, but improvement of the enzymatic hydrolysis is still considered one of the main research challenges for achieving cost-effective lignocellulose-to-ethanol processing.

One of the factors limiting the yield of enzymatic hydrolysis is the inhibitory effect of hydrolysis products on the enzyme activity. Such inhibitory effects usually limit hydrolysis reaction rate and yield, as a global result of complex interactions between the ligno-cellulosic substrate and the enzymes.

Inhibitory effects are more evident when hydrolysis is conducted at low enzyme loading and at high consistency, both conditions corresponding to the most interesting situation from an industrial point of view, due to the need to reduce costs associates to enzymes, process and equipment.

A strategy known in the art for reducing the product inhibition effects is to remove a portion solubilized hydrolysis products from the hydrolysis environment to lower inhibitors concentration. In many case, a supplement of fresh enzymes is often adopted, so as to replace soluble enzymes which are also removed.

It is also known in the art that different mono-, di- and oligosaccharides have different inhibitory effects, for instance xylose and glucose, present at the same concentration, produce different hydrolysis yields and rates, and the same result is obtained for instance in the case of xylooligomers and xylose.

It is pointed out that in the prior art there is not a clear and consolidated view of the inhibitory effects of different saccharides, due to the complexity of the hydrolysis reaction.

For instance, in Zhizhuang Xiao et al., "Effects of sugar inhibition on cellulases and β-glucosidase during enzymatic hydrolysis of softwood substrates", Applied Biochemistry and Biotechnology, Vol. 113-116, 2004, pag.1115, it is presented a quantitative approach to determine the inhibition effects of glucose and other sugar monomers during cellulase and β-Glucosidase hydrolysis of two types of cellulosic material. The increased glucose content in the hydrolysate resulted in a dramatic increase in the degrees of inhibition on both β-Glucosidase and cellulase activities. Supplementation of mannose, xylose, and galactose during cellobiose hydrolysis did not show any inhibitory effects on β-glucosidase activity. However, these sugars were shown to have significant inhibitory effects on cellulase activity during cellulose hydrolysis. In the paper it is also shown that inhibitory effects caused by the hemicellulose-derived sugars, which comprise mainly xylooligomers, were less significant than glucose. The study suggests that high-substrate consistency hydrolysis with supplementation of hemicellulose may be a practical solution to minimizing end product inhibition effects while producing hydrolysate with high glucose concentration.

In Youngmi Kim et al., "Soluble inhibitors/deactivators of cellulase enzymes from lignocellulosic biomass", Enzyme and Microbial Technology 48 (2011) 408-415, the inhibitory effects of different soluble sugars and other inhibitory compounds produced in the pretreatment of the ligno-cellulosic biomass are studied. Among soluble sugars, xylo-oligosaccharides were found to be the most important causes of decreased cellulase activity. Soluble xylose sugars, both in oligomeric and monomeric forms, inhibit cellulases instantaneously, an effect that predominates over other inhibitors in decreasing the initial hydrolysis rate of cellulose to glucose.

Despite the complexity of substrate-enzyme interaction, the fundamental base of hydrolysis reaction is that the enzyme must first contact the substrate for the reaction to occur and that an enzyme-substrate interaction must occur before the final product is released. The ligno-cellulosic substrate, which usually has been pretreated, comprises a solid portion which has not been solubilized by the pre-treatment.

The interaction is complicated by the presence of intermediate solubilized reaction products, namely oligosaccharides and disaccharides, and final reaction products, namely monosaccharides. Moreover, mono-, di-, and oligosaccharides may also be present in the pre-treated ligno-cellulosic substrate, as a result of the pre-treatment.

In Pavle Andric et al., "Reactor design for minimizing product inhibition during enzymatic lignocellulose hydrolysis: I. Significance and mechanism of cellobiose and glucose inhibition on cellulolytic enzymes", Biotechnology Advances 28 (2010) 308-324, inhibition mechanism and strategies for limiting products inhibition effects are reviewed. In the paper it is noted that encompassing simple inhibition schemes, at least eight types of reversible inhibition of cellobiose and glucose have been proposed: (i) competitive, (ii) uncompetitive, (iii) non-competitive, (iv) mixed, (v) partial, (vi) substrate, (vii) product, and (viii) allosteric. Competitive inhibition is related to the adsorption of enzymes on the solid portion of the ligno-cellulosic substrate.

Different strategies have been developed so far for reducing the effects competitive inhibition, thereby promoting the adsorption of enzymes on the solid fraction of a pretreated ligno-cellulosic feedstock.

In US2012036768A1, the disclosed methods involve contacting lignocellulosic biomass with an enzyme composition for a period of time at low fiber consistency, and then thickening the mixture and further hydrolyzing the thickened mixture. The thickening can be performed by filtration, optionally reusing the filtrate and/or any enzymes contained therein during the lignocellulose conversion process to increase the efficiency of the process. Hydrolysis of the thickened mixture provides a fermentable sugar mixture having a higher concentration of sugar than fermentable sugar mixtures provided from less concentrated biomass/enzyme mixtures. The disclosed methods have the disadvantage that in the thickening of the mixture a certain amount of solubilized sugars and enzymes are removed from the mixture. Recovering and reusing the solubilized sugars and enzymes from the filtrate may be partial, thereby lowering global hydrolysis yield, and the recovery process adds supplementary costs.

In US8603789 it is disclosed a method for producing glucose from a pretreated ligno-cellulosic feedstock slurry having an undissolved solids content of between about 15 and about 30 wt %, comprising the steps of moving the pretreated feedstock slurry through a pipe; adding cellulase enzyme to the pretreated slurry to produce a slurry comprising cellulase enzyme; dispersing the cellulase enzyme added in the pretreated slurry by using a high shear in-line mixing device, thereby producing a pretreated slurry comprising dispersed cellulase enzyme; and subjecting the pretreated slurry comprising dispersed cellulase enzyme to hydrolysis. Subsequent to high shear in-line mixing of the cellulase enzyme, the pretreated lignocellulosic feedstock slurry comprising dispersed cellulase enzyme may be fed to one or more unmixed hydrolysis reactor, mixed hydrolysis reactor or a combination thereof. The reactors may be operated in batch, continuous, fed-batch modes, or a combination thereof. In the disclosed process, the preferred pretreatment is acid pre-treatment. Sugars produced by the hydrolysis of hemicellulose during acid pretreatment are generally present in the slurry and include xylose, glucose, arabinose, mannose, galactose or a combination thereof. According to one exemplary embodiment, the soluble components of the pretreated feedstock slurry are separated from the solids. Optionally, a washing step may be incorporated into the solids-liquids separation. The separated solids, which contain cellulose, may then be sent to enzymatic hydrolysis with cellulase enzymes in order to convert the cellulose to glucose. The enzymatic hydrolysis of cellulose using cellulase enzymes. The aqueous stream, which includes the sugars released during pretreatment, the pretreatment chemical and other soluble components, may then be fermented using a microorganism capable of fermenting the sugars derived from the hemicellulose component of the feedstock.

In D. Humbird et al., "Process Design and Economics for Biochemical Conversion of Lignocellulosic Biomass to Ethanol Dilute-Acid Pretreatment and Enzymatic Hydrolysis of Corn Stover", Technical Report NREL/TP-5100-47764, May 2011, It is presented a dilute-acid pretreatment, wherein a first stage in pretreatment is a horizontal screw-feed reactor with a short residence time (5-10 minutes), followed by a second stage is a lower temperature/longer residence time "oligomer conversion" step that converts most of the xylose oligomers leaving the first stage to monomeric xylose without generating significant additional degradation products. Optimization of the operating conditions between the two stages met targets with an overall xylan-to-xylose conversion of 79.6% with 6.4% loss to furfural. Remaining oligomers were 9.0%, leaving 5% of the xylan unreacted. Neutralized, diluted hydrolysate is cooled with cooling water at slightly higher than 20 wt % total solids. A 100-hp inline mixer upstream of the continuous hydrolysis reactor mixes the enzyme into the slurry. After the cellulase enzyme stream is mixed in, the total solids loading is 20 wt % (10.6 wt % insoluble) and the temperature is 48°C. Enzyme dosage is 20mg enzyme protein/g cellulose.

US2011312033 discloses spray methods of delivering saccharification enzymes, fermentation organisms, and other hydrolysis or fermentation ingredients onto lignocellulosic biomass. The methods reduce the need for mechanical mixing when the biomass solids are undergoing enzymatic hydrolysis, and reduce dilution to allow higher product titers in the hydrolysis and/or fermentation steps. The methods comprise spraying a liquid comprising one or more saccharification enzymes onto the pretreated biomass composition to form an impregnated biomass composition; and maintaining the impregnated biomass composition under suitable conditions to promote hydrolysis of components in the impregnated biomass composition to produce the saccharification product. More specifically, US2011312033 provides a method of producing a saccharification product from a pretreated biomass composition, the method comprising: a) spraying a liquid comprising one or more saccharification enzymes onto the pretreated biomass composition to form an impregnated biomass composition; and b) maintaining the impregnated biomass composition under suitable conditions to promote hydrolysis of components in the impregnated biomass composition to produce the saccharification product. The liquid may be a prehydrolysate produced from the pretreated biomass composition. While the use of spraying may be helpful to disperse enzymes homogeneously in the pretreated biomass composition at a high dry matter content, it is noted that spray techniques may induce a stress on the enzymes, resulting in at least a partial deactivation of the enzymes.

There is therefore the need to develop an efficient process for the hydrolysis of a pre-treated ligno-cellulosic biomass, wherein the process is preferably conducted in continuous mode for industrial applications on large scale, at low enzyme dosage and high solid consistency.

SUMMARY OF INVENTION

It is disclosed a process for producing a hydrolyzed mixture comprising glucose from a pre-treated ligno-cellulosic slurry comprising a slurry liquid and slurry solids comprised of lignin and water insoluble sugars comprising glucans, wherein the process comprises the steps of:

a. Adsorbing at least a portion of a cellulase from an enzyme cocktail having an initial cellulase activity onto the slurry solids of the pre-treated ligno-cellulosic biomass slurry to create an adsorbed cellulase wherein the adsorbed cellulase has an adsorbed cellulase activity which is greater than 45% of the initial cellulase activity;

b. Subjecting pre-treated ligno-cellulosic slurry to enzymatic hydrolysis to produce the hydrolyzed mixture.

It is also disclosed that the pre-treated ligno-cellulosic slurry liquid may comprise water soluble sugar oligomers.

It is further disclosed that the concentration of the water soluble sugars in the pre-treated ligno-cellulosic slurry may be greater than zero and less than a value selected from the group consisting of 55g/Kg, 50g/Kg, 40Kg/Kg, and 30g/Kg of the pre-treated ligno-cellulosic slurry.

It is also disclosed that the concentration of the water soluble sugars in the pre-treated ligno-cellulosic slurry divided by the concentration of the water insoluble sugars in the pre-treated ligno-cellulosic slurry may be a value in the range of 0.1 to 0.65, 0.1 to 0.5, 0.1 to 0.4, 0.1 to 0.3, 0.1 to 0.2,0.2 to 0.65, 0.3 to 0.65, 0.4 to 0.65, and 0.5 to 0.65.

It is further disclosed that the pre-treated ligno-cellulosic slurry may further comprise a concentration of water soluble sugar oligomers and the concentration of water soluble oligomers in the pre-treated ligno-cellulosic slurry may be greater than zero and less than a value selected from the group consisting of 15g/Kg, 10g/Kg, 7.5g/Kg, 2.5g/Kg and 5g/Kg of the pre-treated ligno-cellulosic slurry.

It is also disclosed that the water soluble sugar oligomers may comprise xylooligomers, and the percent amount of xylooligomers by weight may be at least a value selected from the group consisting of 70, 75, 80, 85 and 90 percent of the total amount of the water soluble oligomers in the pre-treated ligno-cellulosic slurry.

It is further disclosed that the pre-treated ligno-cellulosic slurry may further comprise a glucose concentration, and the concentration of glucose in the pre-treated ligno-cellulosic slurry may be less than a value selected from the group consisting of 15 g/Kg, 10 g/Kg and 5 g/Kg of the pre-treated ligno-cellulosic slurry.

It is further disclosed that the pre-treated ligno-cellulosic slurry may further comprise a concentration of xylose, and the concentration of xylose in the pre-treated ligno-cellulosic slurry may be less than a value selected from the group consisting of 15 g/Kg, 10 g/Kg and 5 g/Kg of the pre-treated ligno-cellulosic slurry.

It is also disclosed that the pre-treated ligno-cellulosic slurry may further comprise a concentration of cellobiose, and the concentration of cellobiose in the pre-treated ligno-cellulosic slurry may be less than a value selected from the group consisting of 2 g/Kg, 1.5 g/Kg, 1 g/Kg, and 0.5 g/Kg of the pre-treated ligno-cellulosic slurry.

It is further disclosed that the pre-treated ligno-cellulosic slurry may further comprise a concentration of xylobiose, and the concentration of xylobiose in the pre-treated ligno-cellulosic slurry may be less than a value selected from the group consisting of 5.5 g/Kg 5 g/Kg, 4 g/Kg, 3 g/Kg, 2 g/Kg, and 1g/Kg of the pre-treated ligno-cellulosic slurry.

It is also disclosed that the dry matter content of the pre-treated ligno-cellulosic slurry by weight may be greater than a value selected from the group consisting of 10%, 15%, 18%, 20%, 22% and 25%.

It is further disclosed that the enzyme cocktail may further comprise proteins and the total amount of proteins of the enzyme cocktail per gr of glucans in the pre-treated ligno-cellulosic slurry may less than a value selected from the group consisting 10, 7.5, 5, 4, 3, and 2 mg.

It is also disclosed that the adsorbed cellulase activity may be at least a value selected from the group consisting of 50%,55%, 60%, 65% 70%, 75%, 80% and 85% of the initial cellulase activity.

It is further disclosed that the cellulase may be selected from the group consisting of endoglucanase, cellobiohydrolase, beta-glucosidase, and combinations thereof.

It is also disclosed that the enzyme cocktail may further comprise a xylanase having an initial xylanase activity, and that at least a portion of the xylanase may be adsorbed on the solid pre-treated ligno-cellulosic feedstock, so that the adsorbed xylanase has a xylanase activity which is at least a value selected from the group consisting of 40%, 50%, 60%, 70% and 80% of the initial xylanase activity.

It is further disclosed that the slurry solids of the pre-treated ligno-cellulosic slurry may have been homogeneously dispersed in the pre-treated ligno-cellulosic slurry by applying a first shear to the pre-treated ligno-cellulosic slurry.

It is also disclosed that the process may further comprise adjusting the pH of the pre-treated ligno-cellulosic slurry to a value in the range between 4.5 and 5.5, wherein the pH adjusting is done prior to or during applying the first shear to the pre-treated ligno-cellulosic slurry to obtain a homogeneous pH in the pre-treated ligno-cellulosic slurry.

It is further disclosed that a second shear may be applied to the pre-treated ligno-cellulosic slurry for a shearing time after the enzyme cocktail is introduced into the pre-treated ligno-cellulosic slurry or while the enzyme cocktail is being introduced into the pre-treated ligno-cellulosic slurry.

It is also disclosed that the adsorption step may occur during an adsorption time which is greater than 0 seconds and less than a value selected from the group consisting of 5 hours, 1 hour, 40 minutes, 20 minutes, 10 minutes, 5 minutes, and 1 minute.

It is further disclosed that the first shear and the second shear may be applied by means of a unitary shear device comprising more than one shear zone.

It is also disclosed that the shear device may be selected from the group consisting of an homogenizer, a compounder, an extruder, and a mixer.

It is further disclosed that the enzymatic hydrolysis may be conducted in a continuous manner.

It is also disclosed that the enzymatic hydrolysis may comprise the steps of:

a. introducing the pre-treated ligno-cellulosic slurry in a first vessel;
b. Maintaining the pre-treated ligno-cellulosic slurry at hydrolysis conditions to produce a partially hydrolyzed mixture comprising glucooligomers from the glucans of the pre-treated ligno-cellulosic feedstock;
c. removing the partially hydrolyzed mixture from the first vessel.

It is further disclosed that the enzymatic hydrolysis may further comprise the step of hydrolyzing the partially hydrolyzed mixture in at least a second vessel to produce the hydrolyzed mixture.

It is also disclosed that the hydrolysis of the partially hydrolyzed mixture may be conducted in a continuous manner.

It is further disclosed that the hydrolysis of the partially hydrolyzed mixture may be conducted in a batch mode.

BRIEF DESCRIPTION OF FIGURES

Figure 1 is the graph of glucose yield versus time of a batch hydrolysis and a hydrolysis comprising a CSTR step followed by a batch step performed without enzyme adsorption, evidencing the starting problem solved by the disclosed process.

Figure 2 is the graph of the glucans solubilization versus time of the slurry solids which have been pre-hydrolyzed in batch and CSTR configuration (without enzyme adsorption).

Figure 3 is the graph of glucose yield versus time of a batch experiment and two experiments of CSTR step followed by a batch step, performed with and without enzyme adsorption, evidencing the improvement of glucose yield of the disclosed process.

Figure 4 is the graph of glucose yield versus water soluble sugars to water insoluble sugars ratio of representative experiments.

Figure 5 is the graph of glucose yield versus adsorbed endocellulase activity to initial endocellulase activity ratio of representative experiments.

Figure 6 is the graph of glucose yield versus adsorbed xylanase activity to initial xylananase activity ratio of representative experiments.

Figure 7 is the glucose yield of representative experiments performed at different adsorption time.

Figure 8 is the graph of glucose yield versus adsorbed endocellulase activity to initial endocellulase activity ratio of the experiments reported in the experimental section.

Figure 9 is the graph of glucose yield versus adsorbed xylanase activity to initial xylananase activity ratio of the experiments reported in the experimental section.

DETAILED DESCRIPTION

This specification describes a solution to the problem that the continuous enzymatic hydrolysis of pretreated ligno-cellulosic biomass has a lower glucose yield than a batch hydrolysis process. The lower glucose yield is particularly remarkable when the hydrolysis is conducted at a high dry matter content of the pretreated ligno-cellulosic biomass. Namely, at low dry matter content, the glucose yield in a hydrolysis is less affected by the product inhibition, as known in the art. The lower glucose yield is also particularly remarkable when the enzymatic hydrolysis is conducted at a low dosage of the enzyme or enzyme cocktail. Preferred ranges of dry matter and enzymatic cocktail dosage, corresponding to high dry matter and low enzyme dosage, are disclosed in the present specification. Thus, the process disclosed in this specification, which improves the glucose yield of a continuous hydrolysis of pre-treated ligno-cellulosic biomass, is preferably conducted at high dry matter content of the pretreated ligno-cellulosic biomass and at low dosage of the enzyme or enzyme cocktail. These hydrolysis conditions correspond to the most interesting applicative conditions.

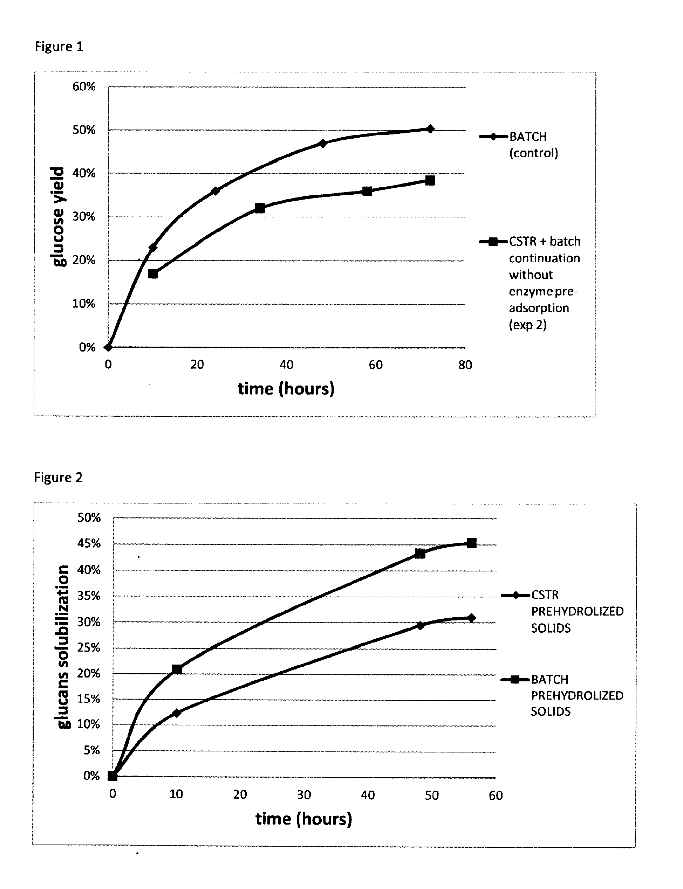

The difference in the glucose yield between a continuous and batch process can be seen in Figure 1. While the continuous process used in Figure 1 is actually a continuous hydrolysis followed by batch hydrolysis, the impact of the continuous process upon the decreased glucose yield is evident.

The solution is based upon the discovery that the glucose yield of a continuous process can be increased by adsorbing at least a portion of the enzymes to the solids of a pretreated ligno-cellulosic biomass prior to introducing the pre-treated ligno-cellulosic biomass with the adsorbed enzymes into the continuous hydrolysis reactor. What inventors found is that, for improving the glucose yield in a continuous process, it is sufficient that the adsorbed enzyme on the solids of a pretreated ligno-cellulosic biomass has an enzymatic activity, called the adsorbed activity, which is at least a certain percentage of the initial activity of the enzyme. The initial enzyme activity is the activity of the enzyme before contacting the pretreated ligno-cellulosic biomass. Based on this discovery, inventors also defined procedures and methods for achieving the desired adsorption of the enzymes to the solids of a pretreated ligno-cellulosic biomass which are particularly suitable for industrial application. Inventors thereby defined a process for adsorbing the enzyme on the solids of a high dry matter content slurry, without any additional concentration step before hydrolysis.

As the experiments demonstrate, the glucose yield increases as the fraction of the relevant activities of the enzymes adsorbed on the solids of the pre-treated ligno-cellulosic biomass.

It is well known to use enzymes for the hydrolysis of pre-treated ligno-cellulosic biomass. An example of the enzymes suitable for the hydrolysis of glucans and xylans of the pre-treated ligno-cellulosic biomass are described in United States Patent Application No. 20130236933, titled "Methods for Producing a Fermentation Product from Lignocellulose-Containing Material, "Methods for Degrading or Converting Cellulosic Materials". This documents contains the following definitions used in this specification.

**DEFINITION**

Cellulolytic Enzyme or Cellulase

The term "cellulolytic enzyme" or "cellulase" means one or more (several) enzymes that hydrolyze a cellulosic material. Such enzymes include endoglucanase(s), cellobiohydrolase(s), beta-glucosidase(s), or combinations thereof.

Endoglucanase

The term "endoglucanase" means an endo-1,4-(1,3;1,4)-beta-D-glucan 4-glucanohydrolase (E.C. 3.2.1.4), which catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components.

Cellobiohydrolase

The term "cellobiohydrolase" means a 1,4-beta-D-glucan cellobiohydrolase (E.C. 3.2.1.91 or E.C. 3.2.1.176), which catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178).

Beta-Glucosidase

The term "beta-glucosidase" means a beta-D-glucoside glucohydrolase (E.C. 3.2.1.21), which catalyzes the hydrolysis of terminal non-reducing beta-D-glucose residues with the release of beta-D-glucose.

Hemicellulolytic enzyme or hemicellulase

The term "hemicellulolytic enzyme" or "hemicellulase" means one or more (several) enzymes that hydrolyze a hemicellulosic material. See, for example, Shallom, D. and Shoham, Y. Microbial hemicellulases. Current Opinion In Microbiology, 2003, 6(3): 219-228). Hemicellulases are key components in the degradation of plant biomass. Examples of hemicellulases include, but are not limited to, an acetylmannan esterase, an acetyxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families marked by numbers. Some families, with overall similar fold, can be further grouped into clans, marked alphabetically (e.g., GH-A). A most informative and updated classification of these and other carbohydrate active enzymes is available on the Carbohydrate-Active Enzymes (CAZy) database.

Xylan Degrading Activity or Xylanolytic Activity

The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (e.g., endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, Recent progress in the assays of xylanolytic enzymes, 2006, Journal of the Science of Food and Agriculture 86(11): 1636-1647; Spanikova and Biely, 2006, Glucuronoyl esterase-Novel carbohydrate esterase produced by Schizophyllum commune, FEBS Letters 580(19): 4597-4601; Herrmann, Vrsanska, Jurickova, Hirsch, Biely, and Kubicek, 1997, The beta-D-xylosidase of Trichoderma reesei is a multifunctional beta-D-xylan xylohydrolase, Biochemical Journal 321: 375-381.

Xylanase

The term "xylanase" means a 1,4-beta-D-xylan-xylohydrolase (E.C. 3.2.1.8) that catalyzes the endohydrolysis of 1,4-beta-D-xylosidic linkages in xylans.

Beta-Xylosidase

The term "beta-xylosidase" means a beta-D-xyloside xylohydrolase (E.C. 3.2.1.37) that catalyzes the exo-hydrolysis of short beta (1→4)-xylooligosaccharides, to remove successive D-xylose residues from the non-reducing termini.

Acetylxylan Esterase

The term "acetylxylan esterase" means a carboxylesterase (EC 3.1.1.72) that catalyses the hydrolysis of acetyl groups from polymeric xylan, acetylated xylose, acetylated glucose, alpha-napthyl acetate, and p-nitrophenyl acetate.

Feruloyl Esterase

The term "feruloyl esterase" means a 4-hydroxy-3-methoxycinnamoyl-sugar hydrolase (EC 3.1.1.73) that catalyzes the hydrolysis of the 4-hydroxy-3-methoxycinnamoyl (feruloyl) group from an esterified sugar, which is usually arabinose in "natural" substrates, to produce ferulate (4-hydroxy-3-methoxycinnamate). Feruloyl esterase is also known as ferulic acid esterase, hydroxycinnamoyl esterase, FAE-III, cinnamoyl ester hydrolase, FAEA, cinnAE, FAE-I, or FAE-II.

Alpha-Glucuronidase

The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol.

Alpha-L-Arabinofuranosidase

The term "alpha-L-arabinofuranosidase" means an alpha-L-arabinofuranoside arabinofuranohydrolase (EC 3.2.1.55) that catalyzes the hydrolysis of terminal noh-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase.

The enzymatic activity of the relevant enzymes is measured according to the procedures contained in the experimental section of this specification.

Often the enzyme is present in what is called an enzyme cocktail, or enzyme composition, or enzyme mixture, which means that there is more than one enzyme present. In general, a typical enzyme cocktail used in the described process will comprise at least one cellulase enzyme, or comprise a cellulase enzyme. In many cases, the enzyme cocktail will comprise endoglucanase(s), cellobiohydrolase(s), beta-glucosidase(s), or combinations thereof.

The enzyme composition can comprise any protein useful in degrading or converting the cellulosic material.

In one aspect, the enzyme composition comprises or further comprises one or more (e.g., several) proteins/polypeptides selected from the group consisting of a cellulase, a hemicellulase, an esterase, an expansin, a laccase, a ligninolytic enzyme, a pectinase, a peroxidase, a protease, and a swollenin. In another aspect, the cellulase is preferably one or more (e.g., several) enzymes selected from the group consisting of an endoglucanase, a cellobiohydrolase, and a beta-glucosidase. In another aspect, the hemicellulase is preferably one or more (e.g., several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase.

In another aspect, the enzyme composition comprises one or more (e.g., several) cellulolytic enzymes. In another aspect, the enzyme composition comprises or further comprises one or more (e.g., several) hemicellulolytic enzymes. In another aspect, the enzyme composition comprises one or more (e.g., several) cellulolytic enzymes and one or more (e.g., several) hemicellulolytic enzymes. In another aspect, the enzyme composition comprises one or more (e.g., several) enzymes selected from the group of cellulolytic enzymes and hemicellulolytic enzymes. In another aspect, the enzyme composition comprises an endoglucanase. In another aspect, the enzyme composition comprises a cellobiohydrolase. In another aspect, the enzyme composition comprises a beta-glucosidase. In another aspect, the enzyme composition comprises a polypeptide having cellulolytic enhancing activity. In another aspect, the enzyme composition comprises an endoglucanase and a polypeptide having cellulolytic enhancing activity. In another aspect, the enzyme composition comprises a cellobiohydrolase and a polypeptide having cellulolytic enhancing activity. In another aspect, the enzyme composition comprises a beta-glucosidase and a polypeptide having cellulolytic enhancing activity. In another aspect, the enzyme composition comprises an endoglucanase and a cellobiohydrolase. In another aspect, the enzyme composition comprises an endoglucanase and a beta-glucosidase. In another aspect, the enzyme composition comprises a cellobiohydrolase and a beta-glucosidase. In another aspect, the enzyme composition comprises an endoglucanase, a cellobiohydrolase, and a polypeptide having cellulolytic enhancing activity. In another aspect, the enzyme composition comprises an endoglucanase, a beta-glucosidase, and a polypeptide having cellulolytic enhancing activity. In another aspect, the enzyme composition comprises a cellobiohydrolase, a beta-glucosidase, and a polypeptide having cellulolytic enhancing activity. In another aspect, the enzyme composition comprises an endoglucanase, a cellobiohydrolase, and a beta-glucosidase. In another aspect, the enzyme composition comprises an endoglucanase, a cellobiohydrolase, a beta-glucosidase, and a polypeptide having cellulolytic enhancing activity.

In another aspect, the enzyme composition comprises an acetylmannan esterase. In another aspect, the enzyme composition comprises an acetylxylan esterase. In another aspect, the enzyme composition comprises an arabinanase (e.g., alpha-L-arabinanase). In another aspect, the enzyme composition comprises an arabinofuranosidase (e.g., alpha-L-arabinofuranosidase). In another aspect, the enzyme composition comprises a coumaric acid esterase. In another aspect, the enzyme composition comprises a feruloyl esterase. In another aspect, the enzyme composition comprises a galactosidase (e.g., alpha-galactosidase and/or beta-galactosidase). In another aspect, the enzyme composition comprises a glucuronidase (e.g., alpha-D-glucuronidase). In another aspect, the enzyme composition comprises a glucuronoyl esterase. In another aspect, the enzyme composition comprises a mannanase. In another aspect, the enzyme composition comprises a mannosidase (e.g., beta-mannosidase). In another aspect, the enzyme composition comprises a xylanase. In a preferred aspect, the xylanase is a Family 10 xylanase. In another aspect, the enzyme composition comprises a xylosidase (e.g., beta-xylosidase).

In another aspect, the enzyme composition comprises an esterase. In another aspect, the enzyme composition comprises an expansin. In another aspect, the enzyme composition comprises a laccase. In another aspect, the enzyme composition comprises a ligninolytic enzyme. In a preferred aspect, the ligninolytic enzyme is a manganese peroxidase. In another preferred aspect, the ligninolytic enzyme is a lignin peroxidase. In another preferred aspect, the ligninolytic enzyme is a H2O2-producing enzyme. In another aspect, the enzyme composition comprises a pectinase. In another aspect, the enzyme composition comprises a peroxidase. In another aspect, the enzyme composition comprises a protease. In another aspect, the enzyme composition comprises a swollenin.

One or more (e.g., several) components of the enzyme composition may be wild-type proteins, recombinant proteins, or a combination of wild-type proteins and recombinant proteins. For example, one or more (e.g., several) components may be native proteins of a cell, which is used as a host cell to express recombinantly one or more (e.g., several) other components of the enzyme composition. One or more (e.g., several) components of the enzyme composition may be produced as monocomponents, which are then combined to form the enzyme composition. The enzyme composition may be a combination of multicomponent and monocomponent protein preparations.

The enzymes used in the processes of the present invention may be in any form suitable for use, such as, for example, a fermentation broth formulation or a cell composition, a cell lysate with or without cellular debris, a semi-purified or purified enzyme preparation, or a host cell as a source of the enzymes. The enzyme composition may be a dry powder or granulate, a non-dusting granulate, a liquid, a stabilized liquid, or a stabilized protected enzyme. Liquid enzyme preparations may, for instance, be stabilized by adding stabilizers such as a sugar, a sugar alcohol or another polyol, and/or lactic acid or another organic acid according to established processes.

The polypeptides having cellulolytic enzyme activity or hemicellulolytic enzyme activity as well as other proteins/polypeptides useful in the degradation of the cellulosic material, can be derived or obtained from any suitable origin, including, bacterial, fungal, yeast, plant, or mammalian origin. The term "obtained" also means herein that the enzyme may have been produced recombinantly in a host organism employing methods described herein, wherein the recombinantly produced enzyme is either native or foreign to the host organism or has a modified amino acid sequence, e.g., having one or more (e.g., several) amino acids that are deleted, inserted and/or substituted, i.e., a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained recombinantly, such as by site-directed mutagenesis or shuffling.

A polypeptide having enzyme activity may be a bacterial polypeptide. For example, the polypeptide may be a Gram-positive bacterial polypeptide such as a Bacillus, Streptococcus, Streptomyces, Staphylococcus, Enterococcus, Lactobacillus, Lactococcus, Clostridium, Geobacillus, Caldicellulosiruptor, Acidothermus, Thermobifidia, or Oceanobacillus polypeptide having enzyme activity, or a Gram negative bacterial polypeptide such as an E. coli, Pseudomonas, Salmonella, Campylobacter, Helicobacter, Flavobacterium, Fusobacterium, Ilyobacter, Neisseria, or Ureaplasma polypeptide having enzyme activity.

In one aspect, the polypeptide is a Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, or Bacillus thuringiensis polypeptide having enzyme activity.

In another aspect, the polypeptide is a Streptococcus equisimilis, Streptococcus pyogenes, Streptococcus uberis, or Streptococcus equi subsp. Zooepidemicus polypeptide having enzyme activity.

In another aspect, the polypeptide is a Streptomyces achromogenes, Streptomyces avermitilis, Streptomyces coelicolor, Streptomyces griseus, or Streptomyces lividans polypeptide having enzyme activity.

The polypeptide having enzyme activity may also be a fungal polypeptide, and more preferably a yeast polypeptide such as a Candida, Kluyveromyces, Pichia, Saccharomyces, Schizosaccharomyces, or Yarrowia polypeptide having enzyme activity; or more preferably a filamentous fungal polypeptide such as an Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella, or Xylaria polypeptide having enzyme activity.

In one aspect, the polypeptide is a Saccharomyces carlsbergensis, Saccharomyces cerevisiae, Saccharomyces diastaticus, Saccharomyces douglasii, Saccharomyces kluyveri, Saccharomyces norbensis, or Saccharomyces oviformis polypeptide having enzyme activity.

In another aspect, the polypeptide is an Acremonium cellulolyticus, Aspergillus aculeatus, Aspergillus awamori, Aspergillus fumigatus, Aspergillus foetidus, Aspergillus japonicus, Aspergillus nidulans, Aspergillus niger, Aspergillus oryzae, Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia spededonium, Thielavia setosa, Thielavia subthermophila, Thielavia terrestris, Trichoderma harzianum, Trichoderma koningii, Trichoderma longibrachiatum, Trichoderma reesei, Trichoderma viride, or Trichophaea saccata polypeptide having enzyme activity.

Chemically modified or protein engineered mutants of polypeptides having enzyme activity may also be used.

One or more (e.g., several) components of the enzyme composition may be a recombinant component, i.e., produced by cloning of a DNA sequence encoding the single component and subsequent cell transformed with the DNA sequence and expressed in a host (see, for example, WO 91/17243 and WO 91/17244). The host is preferably a heterologous host (enzyme is foreign to host), but the host may under certain conditions also be a homologous host (enzyme is native to host). Monocomponent cellulolytic proteins may also be prepared by purifying such a protein from a fermentation broth.

In one aspect, the one or more (e.g., several) cellulolytic enzymes comprise a commercial cellulolytic enzyme preparation. Examples of commercial cellulolytic enzyme preparations suitable for use in the present invention include, for example, CELLIC® CTec (Novozymes A/S), CELLIC® CTec2 (Novozymes A/S), CELLIC® Ctec3 (Novozymes A/S), CELLUCLAST™ (Novozymes A/S), NOVOZYM™ 188 (Novozymes A/S), CELLUZYME™ (Novozymes A/S), CEREFLO™ (Novozymes A/S), and ULTRAFLO™ (Novozymes A/S), ACCELERASE™ (Genencor Int.), LAMINEX™ (Genencor Int.), SPEZYME™ CP (Genencor Int.), FILTRASE® NL (DSM); METHAPLUS® S/L 100 (DSM), ROHAMENT™ 7069 W (Rohm GmbH), FIBREZYME® LDI (Dyadic International, Inc.), FIBREZYME® LBR (Dyadic International, Inc.), or VISCOSTAR® 150 L (Dyadic International, Inc.).

Examples of bacterial endoglucanases that can be used in the processes of the present invention, include, but are not limited to, an Acidothermus cellulolyticus endoglucanase (WO 91/05039; WO 93/15186; U.S. Pat. No. 5,275,944; WO 96/02551; U.S. Pat. No. 5,536,655, WO 00/70031, WO 05/093050); Thermobifida fusca endoglucanase III (WO 05/093050); and Thermobifida fusca endoglucanase V (WO 05/093050).

Examples of fungal endoglucanases that can be used in the present invention, include, but are not limited to, a Trichoderma reesei endoglucanase I (Penttila et al., 1986, Gene 45: 253-263, Trichoderma reesei Cel7B endoglucanase I (GENBANK™ accession no. M15665), Trichoderma reesei endoglucanase II (Saloheimo, et al., 1988, Gene 63:11-22), Trichoderma reesei Cel5A endoglucanase II (GENBANK™ accession no. M19373), Trichoderma reesei endoglucanase III (Okada et al., 1988, Appl. Environ. Microbiol. 64: 555-563, GENBANK™ accession no. AB003694), Trichoderma reesei endoglucanase V (Saloheimo et al., 1994, Molecular Microbiology 13: 219-228, GENBANK™ accession no. Z33381), Aspergillus aculeatus endoglucanase (Ooi et al., 1990, Nucleic Acids Research 18: 5884), Aspergillus kawachii endoglucanase (Sakamoto et al., 1995, Current Genetics 27: 435-439), Erwinia carotovara endoglucanase (Saarilahti et al., 1990, Gene 90: 9-14), Fusarium oxysporum endoglucanase (GENBANK™ accession no. L29381), Humicola grisea var. thermoidea endoglucanase (GENBANK™ accession no. AB003107), Melanocarpus albomyces endoglucanase (GENBANK™ accession no. MAL515703), Neurospora crassa endoglucanase (GENBANK™ accession no. XM-324477), Humicola insolens endoglucanase V, Myceliophthora thermophila CBS 117.65 endoglucanase, basidiomycete CBS 495.95 endoglucanase, basidiomycete CBS 494.95 endoglucanase, Thielavia terrestris NRRL 8126 CEL6B endoglucanase, Thielavia terrestris NRRL 8126 CEL6C endoglucanase, Thielavia terrestris NRRL 8126 CEL7C endoglucanase, Thielavia terrestris NRRL 8126 CEL7E endoglucanase, Thielavia terrestris NRRL 8126 CEL7F endoglucanase, Cladorrhinum foecundissimum ATCC 62373 CEL7A endoglucanase, and Trichoderma reesei strain No. VTT-D-80133 endoglucanase (GENBANK™ accession no. M15665). Examples of cellobiohydrolases useful in the present invention include, but are not limited to, Aspergillus aculeatus cellobiohydrolase II (WO 2011/059740), Chaetomium thermophilum cellobiohydrolase I, Chaetomium thermophilum cellobiohydrolase II, Humicola insolens cellobiohydrolase I, Myceliophthora thermophila cellobiohydrolase II (WO 2009/042871), Thielavia hyrcanie cellobiohydrolase II (WO 2010/141325), Thielavia terrestris cellobiohydrolase II (CEL6A, WO 2006/074435), Trichoderma reesei cellobiohydrolase I, Trichoderma reesei cellobiohydrolase II, and Trichophaea saccata cellobiohydrolase II (WO 2010/057086).

Examples of beta-glucosidases useful in the present invention include, but are not limited to, beta-glucosidases from Aspergillus aculeatus (Kawaguchi et al., 1996, Gene 173: 287-288), Aspergillus fumigatus (WO 2005/047499), Aspergillus niger (Dan et al., 2000, J. Biol. Chem. 275: 4973-4980), Aspergillus oryzae (WO 2002/095014), Penicillium brasilianum IBT 20888 (WO 2007/019442 and WO 2010/088387), Thielavia terrestris (WO 2011/035029), and Trichophaea saccata (WO 2007/019442).

The beta-glucosidase may be a fusion protein. In one aspect, the beta-glucosidase is an Aspergillus oryzae beta-glucosidase variant BG fusion protein (WO 2008/057637) or an Aspergillus oryzae beta-glucosidase fusion protein (WO 2008/057637.

Other useful endoglucanases, cellobiohydrolases, and beta-glucosidases are disclosed in numerous Glycosyl Hydrolase families using the classification according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696.

Other cellulolytic enzymes that may be used in the present invention are described in WO 98/13465, WO 98/015619, WO 98/015633, WO 99/06574, WO 99/10481, WO 99/025847, WO 99/031255, WO 2002/101078, WO 2003/027306, WO 2003/052054, WO 2003/052055, WO 2003/052056, WO 2003/052057, WO 2003/052118, WO 2004/016760, WO 2004/043980, WO 2004/048592, WO 2005/001065, WO 2005/028636, WO 2005/093050, WO 2005/093073, WO 2006/074005, WO 2006/117432, WO 2007/071818, WO 2007/071820, WO 2008/008070, WO 2008/008793, U.S. Pat. No. 5,457,046, U.S. Pat. No. 5,648,263, and U.S. Pat. No. 5,686,593.

In one aspect, the one or more (e.g., several) hemicellulolytic enzymes comprise a commercial hemicellulolytic enzyme preparation. Examples of commercial hemicellulolytic enzyme preparations suitable for use in the present invention include, for example, SHEARZYME™ (Novozymes A/S), CELLIC® HTec (Novozymes A/S), CELLIC® HTec2 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE®XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK).

Examples of xylanases useful in the processes of the present invention include, but are not limited to, xylanases from Aspergillus aculeatus (GeneSeqP:AAR63790; WO 94/21785), Aspergillus fumigatus (WO 2006/078256), Penicillium pinophilum (WO 2011/041405), Penicillium sp. (WO 2010/126772), Thielavia terrestris NRRL 8126 (WO 2009/079210), and Trichophaea saccata GH10 (WO 2011/057083).

Examples of beta-xylosidases useful in the processes of the present invention include, but are not limited to, beta-xylosidases from Neurospora crassa (SwissProt accession number Q7SOW4), Trichoderma reesei (UniProtKB/TrEMBL accession number Q92458), and Talaromyces emersonii (SwissProt accession number Q8×212).

Examples of acetylxylan esterases useful in the processes of the present invention include, but are not limited to, acetylxylan esterases from Aspergillus aculeatus (WO 2010/108918), Chaetomium globosum (Uniprot accession number Q2GWX4), Chaetomium gracile (GeneSeqP accession number AAB82124), Humicola insolens DSM 1800 (WO 2009/073709), Hypocrea jecorina (WO 2005/001036), Myceliophtera thermophila (WO 2010/014880), Neurospora crassa (UniProt accession number q7s259), Phaeosphaeria nodorum (Uniprot accession number QOUHJ1), and Thielavia terrestris NRRL 8126 (WO 2009/042846).

Examples of feruloyl esterases (ferulic acid esterases) useful in the processes of the present invention include, but are not limited to, feruloyl esterases form Humicola insolens DSM 1800 (WO 2009/076122), Neosartorya fischeri (UniProt Accession number A1D9T4), Neurospora crassa (UniProt accession number Q9HGR3), Penicillium aurantiogriseum (WO 2009/127729), and Thielavia terrestris (WO 2010/053838 and WO 2010/065448).

Examples of arabinofuranosidases useful in the processes of the present invention include, but are not limited to, arabinofuranosidases from Aspergillus niger (GeneSeqP accession number AAR94170), Humicola insolens DSM 1800 (WO 2006/114094 and WO 2009/073383), and M. giganteus (WO 2006/114094).

Examples of alpha-glucuronidases useful in the processes of the present invention include, but are not limited to, alpha-glucuronidases from Aspergillus clavatus (UniProt accession number alccl2), Aspergillus fumigatus (SwissProt accession number Q4WW45), Aspergillus niger (Uniprot accession number Q96WX9), Aspergillus terreus (SwissProt accession number QOCJP9), Humicola insolens (WO 2010/014706), Penicillium aurantiogriseum (WO 2009/068565), Talaromyces emersonii (UniProt accession number Q8×211), and Trichoderma reesei (Uniprot accession number Q99024).

The polypeptides having enzyme activity used in the processes of the present invention may be produced by fermentation of the above-noted microbial strains on a nutrient medium containing suitable carbon and nitrogen sources and inorganic salts, using procedures known in the art (see, e.g., Bennett, J. W. and LaSure, L. (eds.), More Gene Manipulations in Fungi, Academic Press, CA, 1991). Suitable media are available from commercial suppliers or may be prepared according to published compositions (e.g., in catalogues of the American Type Culture Collection). Temperature ranges and other conditions suitable for growth and enzyme production are known in the art (see, e.g., Bailey, J. E., and 011 is, D. F., Biochemical Engineering Fundamentals, McGraw-Hill Book Company, NY, 1986).

For the purpose of the disclosed process, the enzyme cocktail is usually characterized by the amount of total protein per unit volume or mass and by its enzymatic activity which is the moles of substrate converted by the enzyme per unit time. The enzymatic activity can be related to a specific substrate. For example, the ability to convert cellulose to glucose is the enzyme cocktail's cellulase activity.

Activities are measured at two different points in the process. The first activity is the initial activity, e.g. initial cellulase activity, and is the activity of the enzyme cocktail on a reference substrate prior to exposure to the pretreated ligno-cellulosic biomass. The second activity is the activity to the same reference substrate after adsorption, which is the activity of the enzymes which have been adsorbed onto the solids of the ligno-cellulosic biomass.

For the purposes of this specification, adsorbed cellulase are those cellulase enzymes which have been adsorbed onto the solids of the ligno-cellulosic biomass. It is known in the art that enzymes may form different kind of bonds with the surface of a solid substrate. Adsorbed cellulase are cellulase enzymes which are not present in the liquid portion of the slurry. Thereby, the amount of adsorbed enzyme is defined as the amount of total proteins which are not retrieved in the liquid of the slurry. This adsorbed cellulase will have an adsorbed cellulase activity. Determination of the amount of adsorbed enzyme and its activity is set forth according to the protocol reported in the experimental section.

The process utilizes a pre-treated ligno-cellulosic biomass stream. The methods to create a pre-treated ligno-cellulosic biomass stream from a ligno-cellulosic biomass are well known in the art. A preferred pre-treatment includes steam explosion of the ligno-cellulosic biomass.

Lignocellulose-Containing Material

The term "lignocellulose" or "lignocellulose-containing material" or "lignocellulosic material" or "cellulosic material" or "ligno-cellulosic biomass", means any material containing cellulose. The predominant polysaccharide in the primary cell wall of biomass Is cellulose and the second most abundant is hemicellulose. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

A preferred ligno-cellulosic biomass may be selected from the group consisting of the grasses and woods. Another preferred ligno-cellulosic biomass can be selected from the group consisting of the plants belonging to the conifers, angiosperms and Poaceae families.

Cellulose is generally found, for example, in the stems, leaves, hulls, husks, and cobs of plants or leaves, branches, and wood of trees. The cellulosic material can be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor), pp. 105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp. 23-40, Springer-Verlag, New York). It is understood herein that the cellulose may be in the form of lignocellulose, a plant cell wall material containing lignin, cellulose, and hemicellulose in a mixed matrix. In a preferred aspect, the cellulosic material is any biomass material. In another preferred aspect, the cellulosic material is lignocellulose, which comprises cellulose, hemicelluloses, and lignin.

In one aspect, the cellulosic material is agricultural residue. In another aspect, the cellulosic material is herbaceous material (including energy crops). In another aspect, the cellulosic material is municipal solid waste. In another aspect, the cellulosic material is pulp and paper mill residue. In another aspect, the cellulosic material is waste paper. In another aspect, the cellulosic material is wood (including forestry residue).

In another aspect, the cellulosic material is Arundo, preferably Arundo Donax. In another aspect, the cellulosic material is bagasse, preferably sugar cane bagasse. In another aspect, the cellulosic material is bamboo. In another aspect, the cellulosic material is corn cob. In another aspect, the cellulosic material is corn fiber. In another aspect, the cellulosic material is corn stover. In another aspect, the cellulosic material is miscanthus, preferably miscanthus giganteus. In another aspect, the cellulosic material is orange peel. In another aspect, the cellulosic material is rice straw. In another aspect, the cellulosic material is switchgrass. In another aspect, the cellulosic material is wheat straw.

In another aspect, the cellulosic material is aspen. In another aspect, the cellulosic material is eucalyptus. In another aspect, the cellulosic material is fir. In another aspect, the cellulosic material is pine. In another aspect, the cellulosic material is poplar. In another aspect, the cellulosic material is spruce. In another aspect, the cellulosic material is willow.

In another aspect, the cellulosic material is algal cellulose. In another aspect, the cellulosic material is bacterial cellulose. In another aspect, the cellulosic material is cotton linter. In another aspect, the cellulosic material is filter paper. In another aspect, the cellulosic material is microcrystalline cellulose. In another aspect, the cellulosic material is phosphoric-acid treated cellulose.

In another aspect, the cellulosic material is an aquatic biomass. As used herein the term "aquatic biomass" means biomass produced in an aquatic environment by a photosynthesis process. The aquatic biomass can be algae, emergent plants, floating-leaf plants, or submerged plants.

The cellulosic material may be used as is or may be subjected to pretreatment, using conventional methods known in the art, as described herein. In a preferred aspect, the cellulosic material is pretreated.

In a preferred embodiment, the lignocellulose-containing material is selected from corn stover, corn cobs, corn fiber, switch grass, wheat straw, rice straw, sugar cane bagasse, and algae, and the combination thereof.

PREFERABLE PRETREATMENT

It has been theorized that pretreatment of the feedstock is a solution to the challenge of processing an insoluble solid feedstock comprising lignin or polysaccharides in a pressurized environment. According to US 2011/0312051, sizing, grinding, drying, hot catalytic treatment and combinations thereof are suitable pretreatment of the feedstock to facilitate the continuous transporting of the feedstock. While not presenting any experimental evidence, US 2011/0312051 claims that mild acid hydrolysis of polysaccharides, catalytic hydrogenation of polysaccharides, or enzymatic hydrolysis of polysaccharides are all suitable to create a transportable feedstock. US 2011/0312051 also claims that hot water treatment, steam treatment, thermal treatment, chemical treatment, biological treatment, or catalytic treatment may result in lower molecular weight polysaccharides and depolymerized lignins that are more easily transported as compared to the untreated ones. While this may help transport, there is no disclosure or solution to how to pressurize the solid/liquid slurry resulting from the pre-treatment. In fact, as the inventors have learned the conventional wisdom and conventional systems used for pressuring slurries failed when pre-treated ligno-cellulosic biomass feedstock is used.

In the integrated second generation industrial operations, pre-treatment is often used to ensure that the structure of the ligno-cellulosic content is rendered more accessible to the catalysts, such as enzymes, and at the same time the concentrations of harmful inhibitory by-products such as acetic acid, furfural and hydroxymethyl furfural remain substantially low. There are several strategies to achieve increased accessibility, many of which may yet be invented.

The current pre-treatment strategies imply subjecting the ligno-cellulosic biomass material to temperatures between 110-250°C for 1-60 min e.g.:

Hot water extraction

Multistage dilute acid hydrolysis, which removes dissolved material before inhibitory substances are formed Dilute acid hydrolyses at relatively low severity conditions

Alkaline wet oxidation

Steam explosion.

A preferred pretreatment of a naturally occurring ligno-cellulosic biomass includes a soaking of the naturally occurring ligno-cellulosic biomass feedstock and a steam explosion of at least a part of the soaked naturally occurring ligno-cellulosic biomass feedstock.

The soaking occurs in a substance such as water in either vapor form, steam, or liquid form or liquid and steam together, to produce a product. The product is a soaked biomass containing a first liquid, with the first liquid usually being water in its liquid or vapor form or some mixture.

This soaking can be done by any number of techniques that expose a substance to water, which could be steam or liquid or mixture of steam and water, or, more in general, to water at high temperature and high pressure. The temperature should be in one of the following ranges: 145 to 165°C, 120 to 210°C, 140 to 210°C, 150 to 200°C, 155 to 185°C, 160 to 180°C. Although the time could be lengthy, such as up to but less than 24 hours, or less than 16 hours, or less than 12 hours, or less than 9 hours, or less than 6 hours; the time of exposure is preferably quite short, ranging from 1 minute to 6 hours, from 1 minute to 4 hours, from 1 minute to 3 hours, from 1 minute to 2.5 hours, more preferably 5 minutes to 1.5 hours, 5 minutes to 1 hour, 15 minutes to 1 hour.

If steam is used, it is preferably saturated, but could be superheated. The soaking step can be batch or continuous, with or without stirring. A low temperature soak prior to the high temperature soak can be used. The temperature of the low temperature soak is in the range of 25 to 90°C. Although the time could be lengthy, such as up to but less than 24 hours, or less than 16 hours, or less than 12 hours, or less than 9 hours or less than 6 hours; the time of exposure is preferably quite short, ranging from 1 minute to 6 hours, from 1 minute to 4 hours, from 1 minute to 3 hours, from 1 minute to 2.5 hours, more preferably 5 minutes to 1.5 hours, 5 minutes to 1 hour, 15 minutes to 1 hour.

Either soaking step could also include the addition of other compounds, e.g. $H_2SO4$, $NH_3$, in order to achieve higher performance later on in the process. However, it is preferred that acid, base or halogens not be used anywhere in the process or pre-treatment. The feedstock is preferably void of added sulfur, halogens, or nitrogen. The amount of sulfur, if present, in the composition is in the range of 0 to 1% by dry weight of the total composition. Additionally, the amount of total halogens, if present, are in the range of 0 to 1% by dry weight of the total composition. By keeping halogens from the feedstock, there are no halogens in the lignin conversion products.

The product comprising the first liquid is then passed to a separation step where the first liquid is separated from the soaked biomass. The liquid will not completely separate so that at least a portion of the liquid is separated, with preferably as much liquid as possible in an economic time frame. The liquid from this separation step is known as the first liquid stream comprising the first liquid. The first liquid will be the liquid used in the soaking, generally water and the soluble species of the feedstock. These water soluble species are glucan, xylan, galactan, arabinan, glucolygomers, xyloolygomers, galactolygomers and arabinolygomers. The solid biomass is called the first solid stream as it contains most, if not all, of the solids.

The separation of the liquid can again be done by known techniques and likely some which have yet to be invented. A preferred piece of equipment is a press, as a press will generate a liquid under high pressure.

The first solid stream is then steam exploded to create a steam exploded stream, comprising solids and a second liquid. Steam explosion is a well-known technique in the biomass field and any of the systems available today and in the future are believed suitable for this step.

The described process is to producing a mixture of hydrolyzed sugars, or a hydrolyzed mixture from a pre-treated lignocellulosic slurry. The hydrolyzed mixture will comprise glucose, and may further comprise xylose and other monomeric sugars. The glucose will come mainly from the hydrolysis of cellulose in the pre-treated ligno-cellulosic biomass. Some dimers and oligomers of the monomeric sugars may also be present in the hydrolyzed mixture. The pretreated ligno-cellulosic slurry comprises a slurry liquid and slurry solids of the pre-treated ligno-cellulosic biomass. The slurry solids are preferably in in the form of solid particles, and comprise glucans and lignin. They may further comprise xylans and other insoluble sugars which have not been solubilized in the pre-treatment. In one aspect, the pre-treated ligno-cellulosic biomass is present in a slurry form as the result of the pre-treatment. In another aspect, the pre-treated ligno-cellulosic biomass is typically in solid particles, and it is typically slurried into a ligno-cellulosic biomass slurry before beginning the adsorption of the cellulase onto the solid portion of the ligno-cellulosic biomass slurry. In some embodiments, the enzymatic cocktail comprising the cellulase will be brought into contact with the ligno-cellulosic biomass during the creation of the ligno-cellulosic biomass slurry. For example, the enzyme cocktail could be added to the slurry liquid, which preferentially comprises water and water soluble sugars, prior to adding the slurry liquid to the ligno-cellulosic biomass during the creation of the ligno-cellulosic biomass slurry. Another embodiment is to add the enzyme cocktail simultaneously with the contacting of the slurry liquid with the ligno-cellulosic biomass.

Inventors have found that the enzyme cocktail should not be added directly to the solid ligno-cellulosic biomass prior to slurrying, especially when a low enzymatic dosage is used.

The dry matter content of the pre-treated ligno-cellulosic slurry by weight is preferably greater than a value selected from the group consisting of 10%, 15%, 18%, 20%, 22% and 25%, but less than 100%.

In one embodiment the slurry liquid comprises water and does not contain any water soluble sugars. In another embodiment the slurry liquid further comprises water soluble sugars, which comprise sugar monomers, sugar dimers and sugar oligomers. Sugar monomers include glucose and xylose. Sugar dimers include cellobiose and xylobiose, soluble sugar oligomers includes soluble glucooligomers different from glucose and cellobiose and soluble xylooligomers different from xylose and xylobiose. The water soluble sugars and sugar oligomers may be derived from the pretreatment of a ligno-cellulosic biomass which may or may be not be the same pre-treatment process used to create the pre-treated ligno-cellulosic biomass for the claimed process.

Inventors found that when the water soluble sugar concentration in the slurry is above a certain amount the adsorption of the enzyme on solids decreases.

Therefore, it is believed that there are preferable concentrations of water soluble sugars in the slurry, which allow the adsorbed enzyme activity to be greater than 45%. These preferable concentration can be expressed in different ways, for example:

- the concentration of the water soluble sugars in the pre-treated ligno-cellulosic slurry is greater than zero and less than a value selected from the group consisting of 55g/Kg, 50g/Kg, 40Kg/Kg, and 30g/Kg of the pre-treated ligno-cellulosic slurry; or
- the concentration of the water soluble sugars in the pre-treated ligno-cellulosic slurry divided by the concentration of the water insoluble sugars in the pre-treated ligno-cellulosic slurry is value in the range of 0.1 to 0.65, 0.1 to 0.5, 0.1 to 0.4, 0.1 to 0.3, 0.1 to 0.2, 0.2 to 0.65, 0.3 to 0.65, 0.4 to 0.65, and 0.5 to 0.65; or
- the concentration of water soluble oligomers in the pre-treated ligno-cellulosic slurry is greater than zero and less than a value selected from the group consisting of 15g/Kg, 10g/Kg, 7.5g/Kg, 2.5g/Kg and 5g/Kg of the pre-treated ligno-cellulosic slurry; or
- the percent amount of xylooligomers by weight is at least a value selected from the group consisting of 70, 75, 80, 85 and 90 percent of the total amount of the water soluble oligomers in the pre-treated ligno-cellulosic slurry; or
- the concentration of glucose in the pre-treated ligno-cellulosic slurry is less than a value selected from the group consisting of 15 g/Kg, 10 g/Kg and 5 g/Kg of the pre-treated ligno-cellulosic slurry; or

- the concentration of xylose in the pre-treated ligno-cellulosic slurry is less than a value selected from the group consisting of 15 g/Kg, 10 g/Kg and 5 g/Kg of the pre-treated ligno-cellulosic slurry; or
- the concentration of cellobiose in the pre-treated ligno-cellulosic slurry is less than a value selected from the group consisting of 2 g/Kg, 1.5 g/Kg, 1 g/Kg, and 1 g/Kg of the pre-treated ligno-cellulosic slurry; or
- the concentration of xylobiose in the pre-treated ligno-cellulosic slurry is less than a value selected from the group consisting of 5.5 g/Kg 5 g/Kg, 4 g/Kg, 3 g/Kg, 2 g/Kg, and 1g/Kg of the pre-treated ligno-cellulosic slurry.

The alternative expressions can also be combined to further restrict the ranges of the preferred concentrations.

As evident from the experimental section, inventors discovered that the enzyme is adsorbed on the slurry solids even in the presence of a certain concentration of water soluble sugars in the pre-treated ligno-cellulosic slurry, and that the activity of the adsorbed enzyme is sufficient to improve the glucose yield of a continuous hydrolysis.

Inventors have found that for improving the glucose yield, it is required that the activity of the adsorbed enzyme, specifically cellulase, will be greater than a certain percentage of the initial enzyme activity Therefore, in the disclosed process the adsorbed cellulase has an adsorbed cellulase activity which is greater than 45% of the initial cellulase activity. Preferably the adsorbed cellulase activity is at least a value selected from the group consisting of 50%,55%, 60%, 65% 70%, 75%, 80% and 85% of the initial cellulase activity.

The cellulase may selected from the group consisting of endoglucanase, cellobiohydrolase, beta-glucosidase, and combinations thereof.

Inventors have found also that in the case that the enzyme cocktail comprises one or more additional enzymes which are not cellulase, the additional enzyme or enzymes may be adsorbed on the slurry solids and that the adsorption may occur even in the presence of water soluble sugars at the concentrations listed above. Moreover, the additional enzyme can be characterized by an initial enzyme activity, and the activity of adsorbed additional enzyme may be a relevant percentage activity of the initial enzyme activity of the additional enzyme.

Examples of additional enzymes that may be included in the enzyme cocktail include, but are not limited to, hemicellulases, xylanases, beta-xylosidases, lytic polysaccharide monooxygenases (AA9/GH61), and oxidoreductases.

In one embodiment, the additional enzyme is a xylanase having an initial xylanase activity, and at least a portion of the xylanase is adsorbed on the solid pre-treated ligno-cellulosic feedstock, so that the adsorbed xylanase has an adsorbed xylanase activity which is at least a value selected from the group consisting of 40%, 50%, 60%, 70% and 80% of the initial xylanase activity.

The advantages offered by the disclosed process are evident in the case that a low enzymatic dosage is used and preferably a continuous hydrolysis process is applied. Therefore, preferably the amount the enzyme cocktail added to the pre-treated ligno-cellulosic slurry corresponds to a total amount of proteins of the enzyme cocktail per gr of glucans in the pre-treated ligno-cellulosic slurry which is less than a value selected from the group consisting 10, 7.5, 5, 4, 3, and 2 mg.

The slurry is preferably a homogenous slurry, meaning that the standard deviation of the average amount of dry matter of a statistically valid number of aliquots of a statistically valid aliquot size sampled from the slurry in a statistically valid manner is less than 20% of the average amount of dry matter, with less than 10% being more preferred. As an example, the maximum standard deviation allowed in the case of an average dry matter content of 20% and a standard deviation less than 20% of the average amount of dry matter, is 4%.

A preferred way to obtain an homogeneous slurry comprises applying a first shear to the slurry. Application of the first shear may be done by means of a shear device selected from the group consisting of an homogenizer, a compounder, an extruder, and a mixer. Non mechanical agents, such as for instance other physical agents including electromagnetic radiation and ultrasounds, may also be used to promote the formation of an homogeneous slurry.

Preferably the pH of the pre-treated ligno-cellulosic slurry has to be adjusted to a value in the range between 4.5 and 5.5 prior to or during applying the first shear to the pre-treated ligno-cellulosic slurry. Inventors have found that it is important to obtain a homogeneous pH in the pre-treated ligno-cellulosic slurry, especially if a low enzymatic dosage is used as in the claimed ranges. The pH is preferably homogenous in the slurry, meaning that the standard deviation of the average pH of a statistically valid number of aliquots of a statistically valid aliquot size sampled from the slurry in a statistically valid manner is less than 20% of the average pH, with less than 10% being more preferred. Stated in other words, it is not necessary that the pH has the same value in the whole pre-treated ligno-cellulosic slurry, provided that the statistical prescription is observed.

Adsorption of the enzymes occurs by adding the enzyme cocktail to the slurry of ligno-cellulosic biomass and maintaining the mixture for an adsorption time at an adsorption temperature in the range of 4°C to 70°C. Enzyme addition may occur continuously or semi continuously.

In a preferred embodiment, a second shear is applied to the pre-treated ligno-cellulosic slurry for a shearing time to promote the adsorption of the enzyme on the slurry solids. Preferably, the second shear is applied after the enzyme cocktail is introduced into the pre-treated ligno-cellulosic, slurry or while the enzyme cocktail is being introduced into the pre-treated ligno-cellulosic slurry. The intensity of second shear is characterized by a shear rate which may be maintained constant or may me varied along the shearing time.

Preferably, the second shear is applied by means of a unitary shear device comprising more than one shear zone.

The time needed for the adsorption step of the disclosed process is called the adsorption time. That is, adsorption time is the time needed to adsorb at least a portion of the cellulase of enzyme cocktail onto the slurry solids of the pre-treated ligno-cellulosic biomass slurry so that the adsorbed cellulase has an adsorbed cellulase activity which is greater than 45% of the initial cellulase activity, preferably at least a value selected from the group consisting of 50%,55%, 60%, 65% 70%, 75%, 80% and 85% of the initial cellulase activity.

The adsorption time is preferably less than a value selected from the group consisting of 5 hours, 1 hour, 40 minutes, 20 minutes, 10 minutes, 5 minutes, and 1 minute. During the adsorption step, some hydrolysis of the pre-treated ligno-cellulosic slurry may occur. Preferably, the effects of the hydrolysis during the adsorption time are limited, meaning by this that less than 20%, preferably less than 10%, more preferably less than 5% of the insoluble sugars of the pre-treated ligno-cellulosic slurry are converted to soluble sugars.

The data show that increasing the shear rate decreases the adsorption time. Therefore it is believed that under extremely high shear conditions, such as those encountered by an extruder or an aggressive in-line homogenizer such as that supplied by FrymaKoruma, Switzerland, the adsorption time can be lowered to as low as 5 seconds. Thus the adsorption time could be in the range of 5sec to 20 minutes, 5sec to 8 minutes, 5sec to 6 minutes, or 5 sec to 5 minutes.

In any event, as this specification teaches that the adsorption time is a function of the intensity of the shear, the practitioner knows how to vary the shear applied to the slurry during adsorption and measure the adsorption as described in the experimental section in order to arrive at the adsorption time needed to achieve the desired adsorption. In a preferred embodiment, the first shear and the second shear are applied by a unitary shear device comprising more than one shear zone. The homogeneous slurry is produced, or homogenized, in a first zone of the unitary device, wherein the first shear is applied, then the enzyme is introduced and adsorbed into a second zone of the unitary device, wherein the second shear is applied. Preferably the unitary shear device is selected from the group consisting of an homogenizer, a compounder, an extruder, and a mixer.

In the disclosed process, the pre-treated ligno-cellulosic biomass is subjected to enzymatic hydrolysis after adsorption step.

In the hydrolysis step, also known as saccharification, the pre-treated ligno-cellulosic slurry is hydrolyzed to break down cellulose to produce the hydrolyzed mixture comprising glucose. The hydrolyzed mixture may further comprise other fermentable sugars derived from the cellulose and/or hemicellulose, such as, cellobiose, xylose, xylulose, arabinose, mannose, galactose, and/or soluble oligosaccharides.

Enzymatic hydrolysis is preferably carried out in a suitable aqueous environment under conditions that can be readily determined by one skilled in the art. In one aspect, hydrolysis is performed under conditions suitable for the activity of the enzymes, i.e., optimal for the enzymes. The hydrolysis can be carried out as a batch, a fed batch or continuous process where the cellulosic material is fed gradually to, for example, an enzyme containing hydrolysis solution.

The saccharification is generally performed in a vessel, such as for example a stirred-tank reactor under controlled pH, temperature, and mixing conditions. Suitable process time, temperature and pH conditions can readily be determined by one skilled in the art. For example, the saccharification can last up to 200 hours, but is typically performed for preferably about 12 to about 120 hours, e.g., about 16 to about 72 hours or about 24 to about 48 hours. The temperature is in the range of preferably about 25° C. to about 70° C, e.g., about 30° C to about 65° C, about 40° C to about 60° C, or about 50° C to about 55° C.

Even if the hydrolysis can be carried out as a batch, a fed batch or continuous process where the cellulosic material is fed gradually to, for example, an enzyme containing hydrolysis solution, in a preferred embodiment, the hydrolysis is conducted in a continuous manner.

In order for the process to run in a continuous manner, it is not necessary that the pre-treated ligno-cellulosic slurry is continuously introduced to the hydrolysis reactor, but it can be introduced at steady aliquots or pulses. Thus there may be moments when there is no pre-treated ligno-cellulosic slurry entering the hydrolysis reactor. But, over time, the mass introduced into the hydrolysis reactor equals the hydrolyzed mixture removed from the hydrolysis reactor. One distinguishing feature between a continuous and a batch process is that, in a continuous process, the hydrolysis is occurring or progressing at the same time that either the pre-treated ligno-cellulosic slurry is introduced into the hydrolysis reactor and/or the hydrolyzed mixture is removed from the hydrolysis reactor. Another way to state this is that the hydrolysis in the hydrolysis reactor occurs while simultaneously, or at the same time, removing at least a portion of the hydrolyzed mixture from the hydrolysis reactor. Such removal is done in a continuous manner which includes an aliquot or pulse removal. The residence time can last up to 200 hours, but is typically performed for preferably about 12 to about 120 hours, e.g., about 16 to about 72 hours or about 24 to about 48 hours.

In another embodiment, the hydrolysis is performed in two or more steps, wherein the first step is conducted in a continuous manner in a first hydrolysis reactor, or vessel. The product of the first hydrolysis step is a partially hydrolyzed mixture, comprising water soluble gluco-oligomers which have been produced from the insoluble glucans of the pre-treated ligno-cellulosic slurry. The residence time in the first reactor can last up to 200 hours, but is typically performed for preferably about 5 to about 100 hours, e.g., about 8 to about 50 hours or about 10 to about 35 hours.

The partially hydrolyzed mixture removed from the first hydrolysis reactor is then introduced into a second hydrolysis reactor, wherein it is further subjected to a second hydrolysis step. In one embodiment, the second hydrolysis step is performed in a continuous manner to produce the hydrolyzed mixture.

In another embodiment, the second hydrolysis step is performed in a batch or fed batch mode to produce the hydrolyzed mixture.

**EXPERIMENTAL**

**Pretreated streams preparation**

Wheat straw was introduced into a continuous reactor and subjected to a soaking treatment at a temperature of 158°C for 65 minutes. The soaked mixture was separated in a soaked liquid and a fraction containing the solid soaked raw material by means of a press. The fraction containing the solid soaked raw material was subjected to steam explosion at a temperature of 200°C for a time of 4 minutes to produce a solid stream.

Soaked liquid was subjected to a concentration step by means of a membrane filtration step, which also removes a portion of acetic acid.

First, soaked liquids were subjected to a preliminary pre-separation step to remove solids, by means of centrifugation and macro filtration (bag filter with filter size of 1 micron). Centrifugation was performed by means of a Alfa Laval CLARA 80 centrifuge at 8000rpm.

The soaked liquid was then subjected to concentration by means of a Alfa Laval 2.5" equipment (membrane code NF99 2517/48), operated at a VCR (Volume Concentration Ratio) of 2.5.

The pre-treatment, including concentration step, produced a soaked liquid and a solid stream in a ratio liquid stream : solid stream by weight of 1:1.

The soaked liquid and the solid stream were used in the enzymatic hydrolysis experiments.

The dry matter of the soaked liquid after concentration was 10%.

pH of the solid stream was 4 and pH of the liquid stream was 4.

Wheat straw, pre-treated at the indicated conditions, is the reference ligno-cellulosic feedstock used for validating the invention. Other ligno-cellulosic feedstocks pre-treated at different conditions were also tested.

**Hydrolysis experiments**

The invention was proven by performing hydrolysis of the pre-treated streams in different configurations. Batch configuration was used as control. CSTR+Batch continuation was used to demonstrate the disclosed process. Double CSTR configuration is a possible real implementation of the hydrolysis on industrial scale, and the configuration was used for showing the improvement of the disclosed process.

In all the experiments, enzyme cocktail Cellic CTEC3 by Novozymes was used. The amount of enzyme cocktail was determined on the basis of milligram of cocktail solution per gram of glucans in the pretreated streams. In the experiments, protein dosage was approximately 3.5 milligram of protein per gram of glucans.

Reference dry matter was 20% for all the experiments, unless otherwise specified. Thereby some water may be added to the stream to reach the desired dry matter. Dry matter may slightly change in each experiment, without affecting the significance of the test.

The hydrolysis performance was evaluated in terms of glucose yield. Glucose yield is the percent ratio of the amount of glucose in the hydrolyzed mixture to the amount of glucans in the pretreated streams, expressed as glucose equivalents. Glucose equivalents were calculated including insoluble glucans, gluco-oligomers, cellobiose and glucose, present in both the solid and liquid of the ligno-cellulosic biomass, taking into account the different molecular weights.

**Batch configuration**

An amount of 10 Kg of solid stream and an amount of liquid stream were inserted and mixed in a batch reactor (horizontal reactor of 40L of total volume). A different amount of liquid was used in the experiments. Unless otherwise specified, liquid stream was added to obtain the reference dry matter of about 20%, which corresponds to a weight ratio solid : liquid of 1:1, with addition of a certain amount of water.

The solid stream and the liquid were mixed at 20 rpm for 90 minutes. During mixing, pH was set to 5 using a KOH solution (2 M) and temperature was raised to 50°C. Enzyme cocktail was then added.

Hydrolysis was performed under mixing and the glucose was analyzed at different time during the hydrolysis.

**CSTR+Batch continuation**

CSTR+Batch continuation includes a first hydrolysis step in continuous conditions followed by a second hydrolysis step in batch conditions.

The Continuously stirred thank reactor (CSTR) was a vertical reactor of 300 L of total volume. In the reactor, mixing was realized by a specific impeller for high viscous slurry (PARAVISC ™) able to run at 27 rpm. Inside the reactor the slurry is recirculated by a membrane pump.

Two different strategies for inserting the solid stream, the liquid stream and the enzyme were considered.

In a first strategy, the solid stream and the liquid stream were inserted into the CSTR reactor separately. Enzyme cocktail was introduced into the reactor separately. Hydrolysis pH was set and maintained to 5 by adding a KOH solution (2 M) to the reactor; hydrolysis temperature was 50°C.

In a second strategy, the solid stream and the liquid stream were mixed to form a slurry. During mixing, pH was set to 5 using a KOH solution (2 M) and temperature was raised to 50°C. Enzyme cocktail was introduced into the slurry before insertion into the reactor. Different procedures for adsorbing the enzyme on the solids of the slurry were used, as detailed in each experiment.

Unless otherwise specified, the flow rate of the solid stream was 4 Kg/h. A different flow rate of liquid stream and water was used in the experiments, separately or in slurry form with the solid stream. The reference dry matter corresponds to 20%.

A stream of the partially hydrolyzed mixture was continuously removed from the reactor to set the desired residence time, according to standard operation of CSTR reactor. After 3 times the residence time, the system was considered at steady condition. Insertion of the streams and enzyme cocktail and removal of the partially hydrolyzed stream were stopped and hydrolysis was continued in batch configuration under stirring. The hydrolysis yield was analyzed at different time during the hydrolysis.

The present configuration was introduced for comparing the batch continuation step and the batch configuration. Namely, the removal of solubilized sugars from the partially hydrolyzed mixture, which are known to inhibit enzymes action, does not permit to compare pure continuous and batch configurations.

**Double CSTR configuration**

The configuration comprises two CSTR reactors in series. In the first step, the pretreated streams were inserted into a first CSTR reactor and subjected to enzymatic hydrolysis with a first residence time as described in the CSTR + batch continuation section, then the stream removed from the first reactor was inserted in a second CSTR reactor, wherein continuous hydrolysis was continued for a second reaction time. No enzyme cocktail was further inserted into the second hydrolysis step. Hydrolysis yields of both the continuous hydrolysis steps were analyzed at steady condition.

**Determination of the enzyme activity of enzymes adsorbed on solid stream**

Quantification of the amount of total and active enzyme during enzymatic hydrolysis of pre-treated material requires the collection of slurry samples. Each slurry sample is subjected to a solid / liquid separation immediately after the collection using a centrifuge equipped with a swing-out rotor. Each sample was centrifuged for 10 minutes at 4000 rpm and 20°C. After centrifugation, liquid fraction and solid fraction of each samples were kept separated and used to quantify the distribution of total protein and of each activity between solid and liquid fraction.

The activity of enzymes present on solids was determined according to the following protocols.

**Total protein and enzyme activities quantification in the liquid fraction**

Liquid fractions separated from sample slurries were dialyzed for 19 hours using a 8 kDa dialysis membrane tubing (Spectra/Por® 6, 734-0651) against 50mM Tris-HCl buffer pH 5.2 containing 100 mM NaCl. Dialyzed samples were recovered from the dialysis tube and concentrated 10 times using a Vivaspin tube with a 10 kDa cut-off (Vivaspin 512-4050).

Soluble protein concentration was quantified on the concentrated liquid sample by Bradford reagent (Bio-rad). Protein concentration was used to calculate the total amount of protein in the original slurry sample considering dilution, concentration factors and the volume of liquid fraction collected after solid/liquid separation.

The total amount of soluble protein into the liquid fraction stand then for the amount of proteins not adsorbed onto the solid in the hydrolysis slurry and could be used to calculate the fraction of the proteins not adsorbed onto the solid with respect to the total protein expected in the slurry sample. An estimation of the fraction of proteins adsorbed onto the solid fraction could be also obtained as the completion of the previous fraction:

$$Bp, \% = 100 - ( Lp / Ip)$$

where Bp = estimated adsorbed protein expressed in percentage;
Lp= total protein in the liquid fraction (mg);
Ip= total protein expected in the slurry sample (mg)

Enzyme activity quantification was also carried out on the concentrated liquid sample using industry known methods. Filter paper activity (FPU), xylanase and Beta-glucosidase activities were quantified using filter paper, xylan from beechwood and salicin respectively as specific substrates. FPU substrate consists 15 mg Whatman No. 1 filter paper strip (0.55 x 3.29 cm). Xylan and salicin were prepared in 50mM sodium citrate buffer at 2% (w/w) and 1% (w/w) concentrations respectively. Total sugars released by enzyme activity during the assays were quantified by DNS reagent and used to calculate the concentration of active enzyme in the liquid fraction expressed as U/ml of active enzymes. U/ml values obtained using the three specific substrates were used to calculate total amount of active Cellulases, xylanases and Beta-glucosidase in the liquid fraction.

The amount of each enzyme activity in the liquid fraction stand for the amount of each active enzyme category not adsorbed onto the solid in the hydrolysis slurry and could be used to calculate the fraction of each enzyme category not adsorbed onto the solid with respect to the amount of each enzyme expected in the slurry sample. The fraction of each enzyme activity adsorbed onto the solid fraction was estimated as a completion of the previous fraction

$$Bxxxt, \% = 100 - ( Lxxx / Ixxx)$$

where Bxxx = estimated bound enzyme activities expressed in percentage;
Lxxx= enzyme activity quantified in the liquid fraction(U/ml);
Ixxx= enzyme activity expected in the slurry sample (U/ml);
Where xxx indicates the enzyme activity of interest

Based on the specific substrate list, Bcel, Bxyl and Bbg value could be calculated to quantify Adsorbed cellulases, xylanases and beta-glucosidases respectively.

**Enzyme activities quantification in the solid fraction**

Solid fractions obtained from slurry samples taken during hydrolysis were re-suspended in 50 mM sodium citrate buffer pH 5.2 at room temperature at a fixed 1:5 ratio and rapidly centrifuged for 10 minutes at 20°C and 4000 rpm. Separated liquid was discarded and solid portion collected was re-suspended and re-collected in the same way once more to completely remove soluble sugars from solid fractions. After the second separation step, a 25 mg sample of washed solid fraction on wet basis was weighted directly in a 1.5 ml-tube in triplicate (ST, sample tubes).

The endo-cellulase, xylanase and Beta-glucosidase activities were quantified using carboxymethyl cellulose (CMC), xylan from beechwood and salicin respectively as specific substrates. Each substrate was prepared at different concentration in 50mM sodium citrate buffer pH 5.2: 2% (w/w) for CMC and xylan from beechwood and 1% (w/w) for salicin.

1.5 ml of specific substrate were added in each ST. An additional sample tube was prepared weighting 25mg of washed solid in a 1.5 ml sample tube and adding 1.5 ml of 50 mM sodium citrate buffer pH 5.2. Three more samples consisting only of 1.5 ml of each specific substrate and one more sample consisting only of 1.5 ml of buffer were also prepared as control tubes (CT).

All ST and CT tubes were mixed gently inverting the tubes and they were incubated for 10 minutes at 50°C in a constant mixing at 1400 rpm. All samples were then centrifuged for 7 minutes at 20°C and 12000 rpm. Separated solid fractions of each tube were discharded and each separated soluble fraction was moved to a clean tube and centrifuged again for 7 minutes at 20°C and 12000 rpm. Each liquid fraction was moved again to clean tubes and used to quantify product formed during the assay. The amount of reducing sugars released from each specific substrate by the enzyme action were determined by 3,5-dinitrosalicylic acid (DNS) reagent using industry known methods of determining soluble enzymatic activity. The amount of adsorbed enzymes actives on each specific substrate was calculated as a density dividing the U of active enzymes (mg of products/min) for the amount (mg) of material used for the assay on dry basis. The density of each enzyme activity was also referred as a ratio of the theoretical enzyme activity density in the slurry sample.

**Examples**

Two samples were prepared and hydrolyzed for highlighting the problem which is solved by the disclosed process.

**Experiment 1 (control):** Solid stream and liquid stream were hydrolyzed in batch configuration for 72 hours. The final glucose yield was 50%. Batch configuration is the control experiment used in the following experiments.

**Experiment 2**: CSTR+Batch configuration. The solid stream and liquid stream were inserted separately into the CSTR. Enzyme cocktail was inserted into the CSTR reactor separated from the pretreated streams. Residence time was 10 hours. Once the continuous step reached the steady condition, the batch step was started and continued for 62 hours. The final glucose yield was 38%, thereby a gap of 12% occurred with respect to experiment 1 (control).

Figure 1 reports the glucose yield versus total hydrolysis time of the two experiments. Glucose yield of batch step (Exp. 2) is plotted starting at 10hours for taking into account the continuous step residence time. It is noted that the glucose yield of continuous hydrolysis at 10hours is only slightly less than the glucose yield of batch configuration at 10hours.

The example highlights the low glucose hydrolysis yield obtained in a continuous hydrolysis, when the enzyme cocktail is introduced into the CSTR reactor separated from the pretreated stream.

An experiment was further conducted to address the effect of the soluble sugars on enzyme adsorption on solids, thereby affecting hydrolysis performance.

**Experiment 3:** The mixture of CSTR step of experiment 2 (residence time of 10 hours) was removed from the CSTR reactor in steady condition and the liquid portion was collected by centrifugation at 9000 rpm/min. The liquid portion, rich in soluble sugars, was added and mixed to fresh solid stream in a ratio of S: L = 0.2 to reach a dry matter of 13%, a reference amount of fresh enzyme was inserted and hydrolysis was performed in standard batch condition. Glucose yield was calculated taking into account the glucose produced from the hydrolysis of the fresh solid stream. The final glucose yield was about 20%, thereby a gap of 30% occurred with respect to experiment 1 (control).

An experiment was conducted for highlighting the different enzyme adsorption the hydrolysis performance of solids occurring in batch and CSTR + batch configurations.

**Experiment 4:** a portion of the partially hydrolyzed solid stream of control experiment 1 was removed from the batch reactor after a hydrolysis time of 10 hours and washed in abundant water to remove soluble sugars and other soluble components. The washing procedure was the following:

- the hydrolyzed mixture was centrifuged at 9000 rpm/min and the separated solids were collected;
- the solids were washed in an amount of water which was 10 times the volume of the solids. Water temperature was 55°C;
- after shaking the solids in water with a multi-shaker for 15 minutes, the solids were separated and collected by centrifugation at 4000 rpm/min.

The mixture of CSTR step of experiment 2 (residence time of 10 hours) was removed from the CSTR reactor in steady condition and the solid portion was separated and washed with the same procedure. The two washed solid streams were inserted into the batch reactor two identical reactors, water was added to reach a dry matter of 8% and hydrolysis was continued in batch configuration for 58 hours. No enzyme was further inserted. Glucans solubilization of both experiments is reported in figure 2, wherein it is noted that the hydrolysis of the batch solid stream has better performance than the hydrolysis of the continuous solid stream. Glucans solubilization is the equivalent of glucose yield, but considering the partially hydrolyzed solid as reference. The final gap of glucans solubilization, between the two batch configurations of experiment 4, is very close to the gap of glucose yield between experiments 1 and 2.

Different experiments were conducted for closing the gap between the batch and CSTR+batch continuation configurations, thereby defining the disclosed process.

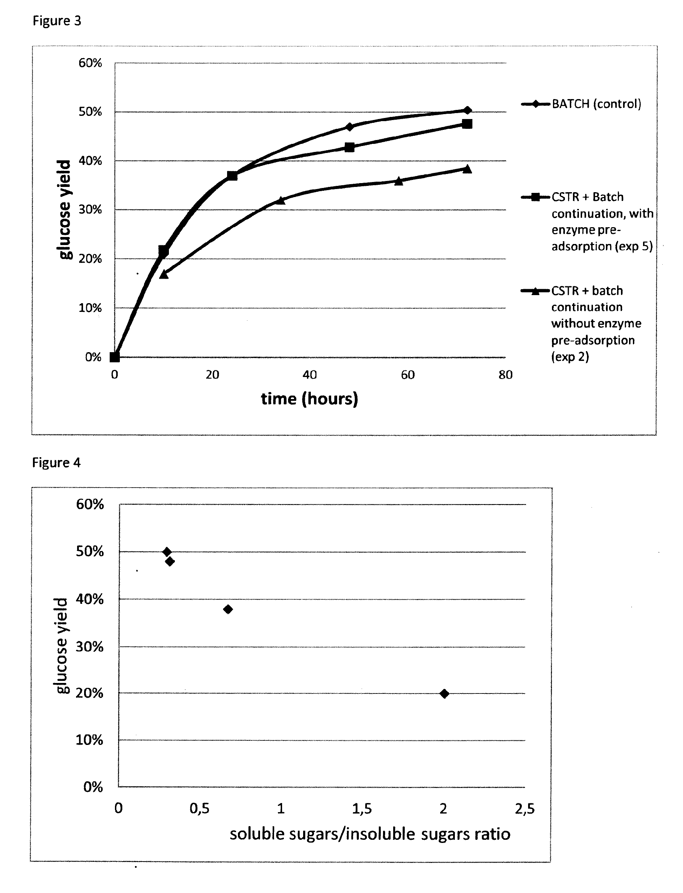

**Experiment 5:** CSTR+batch configuration. A slurry of the solid stream and liquid stream was prepared by mixing the streams to produce a visible homogeneous slurry. During mixing, pH was set to 5 using a KOH solution (2 M) and temperature was raised to 50°C. The enzyme cocktail was then adsorbed on the solids of the slurry in a vessel having a diameter of 40cm by means of a single-arm rectangular impeller having a width of 0.5 cm and rotating around a radius of 15 cm (adsorption procedure 1). Adsorption was performed for an adsorption time of 1 hour at 7 rpm, then the slurry was inserted into the CSTR reactor. Residence time was 10 hours. Once the continuous step reached the steady condition, the batch step was started and continued for 62 hours. Figure 3 reports the glucose yield versus total hydrolysis time of the experiment 5, together with the corresponding plots of experiment 1 and 2.

The final glucose yield was 48%, thereby the gap with respect to experiment 1 (control) was substantially closed and the yield was improved with respect to the CSTR+batch configuration, without enzyme adsorption before hydrolysis.

**Experiment 6:** CSTR + batch configuration. A slurry of the solid stream and liquid stream was prepared and hydrolyzed as in experiment 5, with the exception of the enzyme adsorption time which was reduced to 40 minutes. The final glucose yield was 37%, thereby a gap of 13% occurred with respect to experiment 1 (control).

**Experiment 7:** CSTR + batch configuration. A slurry of the solid stream and liquid stream was prepared and hydrolyzed as in experiment 5, with the exception of the enzyme adsorption time which was reduced to 20 minutes. The final glucose yield was 31%, thereby a gap of 19% occurred with respect to experiment 1 (control).

**Experiment 8:** batch configuration. Solid stream, liquid stream and water corresponding to a dry matter of about 14% were hydrolyzed in batch configuration for 72 hours (as in experiment 1). The final glucose yield was 57%. Batch configuration is the control experiment used in the following experiment 9.

**Experiment 9:** CSTR + batch configuration. Solid stream, liquid stream and water were mixed to have a homogeneous slurry at about 14% DM and then hydrolyzed as in experiment 6 (enzyme adsorption at low shear for 40 minutes). The final glucose yield was 53%, thereby the gap with respect to experiment 8 was substantially closed.

**Experiment 10:** CSTR + batch configuration. A slurry of the solid stream and liquid stream was prepared and hydrolyzed as in experiment 6 with the exception of enzyme adsorption procedure (adsorption procedure 2 in this case). The enzyme was adsorbed by means of a system "BRABENDER" constituted by a vessel with a diameter of 49 cm with two four-arm impellers situated in a rectangular chamber (19.5x30cm) at a height of 29.5 cm from the bottom of the vessel. The arms have a width of 2.5 cm and rotate around a radius of 7.5 cm having a terminal mixing expansion (4x3.5 cm). Adsorption was performed for an adsorption time of 40 minutes at 7 rpm, corresponding to an adsorption condition at a higher shear with respect to the shear of experiment 6, then the slurry was inserted into the CSTR reactor.

The final glucose yield was 47%, thereby the gap with respect to experiment 1 (control) was substantially closed.

**Experiment 11:** double CSTR configuration. The solid stream and liquid stream were inserted separately into the CSTR and subjected to a first continuous hydrolysis step as in experiment 2. At steady conditions, the removed stream from the first continuous reactor was inserted in a second CSTR reactor at a flow rate of 4Kg/hour. The second reactor was identical to the first reactor and was operated in the same operating conditions. No enzyme was added in the second continuous step. The residence time of the second step was 20 hours. At steady conditions, the final glucose yield of the first continuous hydrolysis step was 27%.

**Experiment 12:** Double CSTR configuration. A slurry of the solid stream and liquid stream was prepared and hydrolyzed as in experiment 5. The removed stream was subjected to a second hydrolysis step as in experiment 11. At steady conditions, the final glucose yield was 32 %, showing the improvement of the disclosed process in a continuous hydrolysis.

**Results analysis**

In table 1 the composition of the pretreated streams, glucose yield and enzyme adsorption are reported for each experiment.

Soluble sugars are indicated as single glucose- and xylose-based monomeric, dimeric and oligomeric sugars, and grouped into categories. Compositions of the slurry are given before introducing the enzyme into the slurry. In the case of enzyme introduced separately into the CSTR (exp 2), the composition is relative to the partially hydrolyzed slurry in the CSTR in steady condition, that is the environment wherein the enzyme is inserted into. In exp 4, wherein no fresh enzyme is added to continue the hydrolysis of solids stream coming from a batch and a CSTR step, compositions are omitted. Cellobiose detection limit was 2g/Kg.

As different batches of wheat straw feedstocks were pre-treated and used for demonstrating the disclosed process, slightly different compositions are reported. In the case of exp 5-6- 7-10 the same pre-treated streams were used, thereby the same composition is reported. The same consideration holds for exp8-9.

Activity of enzyme adsorbed on solids in the slurry was measured after adsorbing the enzyme for the adsorption time of each specific experiment, when applicable. In exp 1, 3, 4 and 8, for being consistent with other experiments, activity of adsorbed enzyme was measured after adsorbing the enzyme for 1 hour. A detailed study of the temporal evolution of activity of adsorbed enzyme showed that the adsorbed activities do not change significantly at least in the first hours of hydrolysis. As the enzymatic hydrolysis is characterized by hydrolysis time much longer than adsorption time, it is pointed out that, even if some hydrolysis may occur during the adsorption time, the composition does not change significantly during the enzyme adsorption. In exp 2, activity of adsorbed enzyme was measured on the partially hydrolyzed slurry in the CSTR in steady condition. Representative activities are endocellulase and xylanase. In table 1 it is reported the percent activity ratio of the representative activities adsorbed on solids and the initial activities, measured on the enzyme cocktail. Other activities were also tested on exemplary samples and showed the same trends.

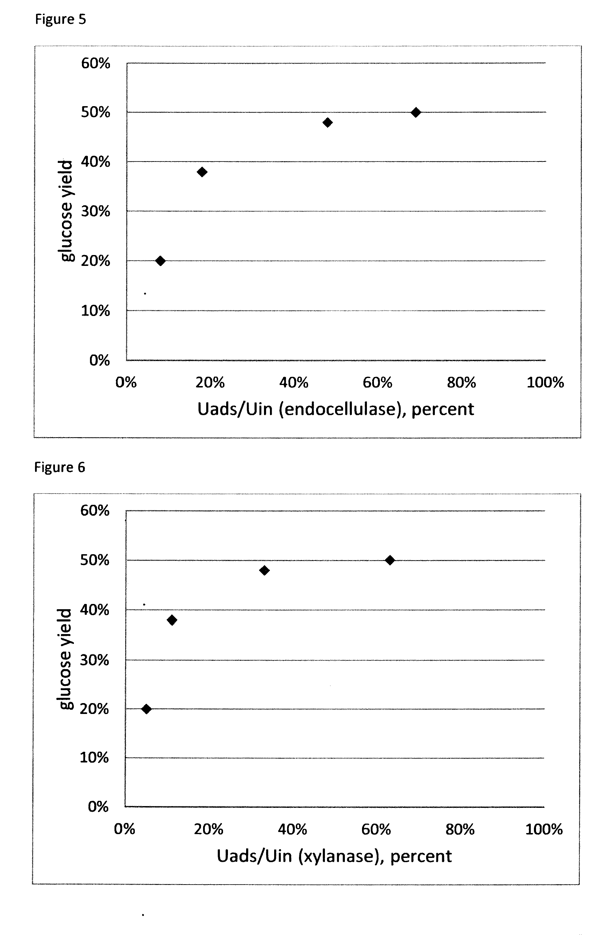

Exp 1-2-3-5 demonstrate a first aspect of the disclosed invention. In exp 1 and 5 the enzyme was added to a slurry characterized by a low ratio of soluble sugars/insoluble sugars and glucose yield was high in both cases despite the fact that enzyme is contacted with the slurry in two different hydrolysis configurations (batch and continuous). In both cases, adsorbed activities were relevant. In exp 2 and 3 the enzyme was added to a slurry characterized by a high ratio of soluble sugars/insoluble sugars and glucose yield was high in both cases. Exp 3 was done in in the presence of soluble sugars from a continuous hydrolysis step, added in a disproportionate amount to emphasize the effect of soluble sugars. In figure 4 it is reported the glucose yield versus soluble sugars to insoluble sugars ratio, evidencing the effect of soluble sugars on active enzyme adsorption. In Figure 5 and 6 it is reported the glucose yield versus the percent activity ratios of endocellulase and xylanase respectively, evidencing the correlation between hydrolysis performance and activity of adsorbed enzyme.

Exp 4 highlights the effect of soluble sugars on adsorbed activities in exp 1 and 2. In exp 2, the enzyme added to the CSTR reactor separately are less actively adsorbed on the solids of the partially hydrolyzed slurry, thereby the hydrolysis continuation occurs in the presence of less enzyme activity and hydrolysis performance is strongly reduced.

Exp 1-6-8-9 highlight the effect of dry matter on glucose yield and adsorbed activities, evidencing that adsorbed activity increases by reducing the dry matter of the slurry, at substantially the same soluble sugars/insoluble sugars ratio. This is proven both in batch configuration (exp 1-8) and in CSTR+batch continuation (exp 6-9).

A second aspect of the disclosed invention is highlighted by comparing the following examples. A suitable enzyme adsorption procedure is required for obtaining a high glucose yield, even in the presence of soluble sugars at a low soluble sugars/insoluble sugars ratio.

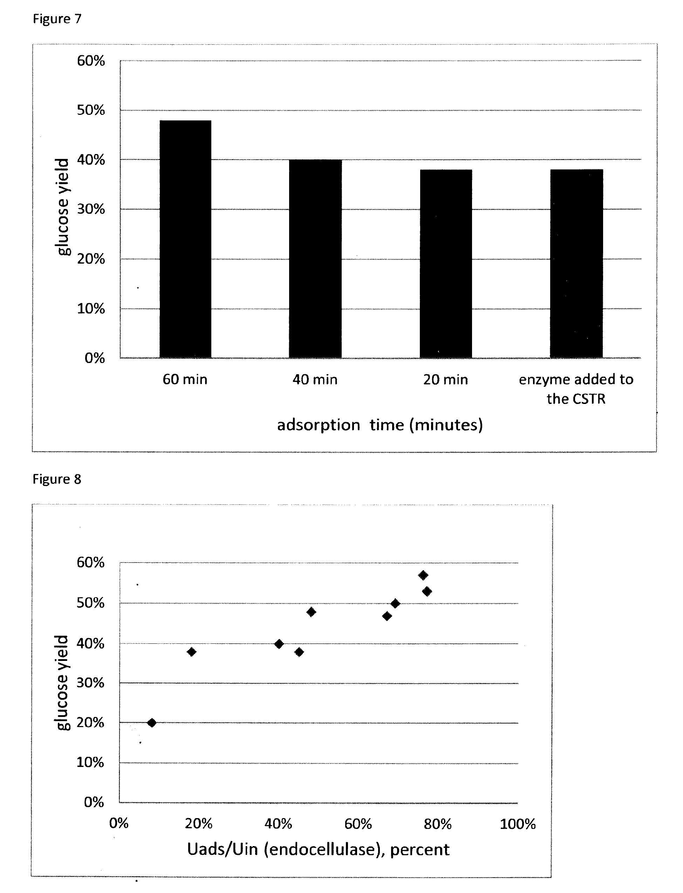

Exp 5-6-7 highlight the effect of adsorption time on glucose yield and adsorbed activities, at the same adsorption procedure and dry matter. In figure 7 the glucose yield of the three experiments and exp 1 is reported, evidencing that at the same adsorption procedure, a certain adsorption time is required to improve hydrolysis yield.

Exp 6-10 highlight the effect of different adsorption procedures on glucose yield and adsorbed activities, at the same adsorption time and dry matter. Increasing the shear intensity, the hydrolysis gap is closed at shorter adsorption time.

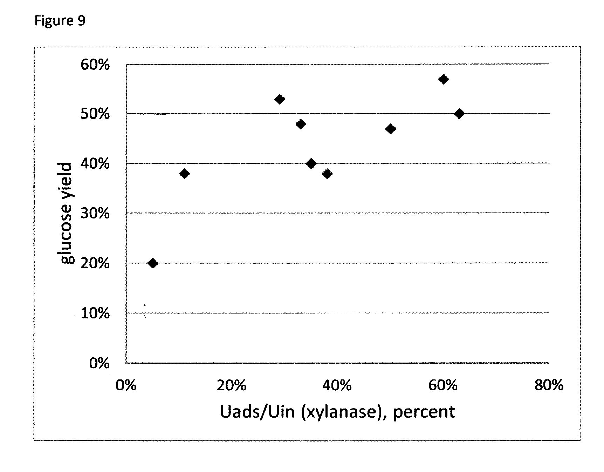

In figure 8 and 9 the glucose yield versus the percent activity ratios of endocellulase and xylanase respectively of all the experiments, evidencing the correlation between hydrolysis performance and activity of adsorbed enzyme. It is noted here that it is sufficient that the adsorbed cellulase activity will be greater than 45% of the initial cellulase activity to improve the glucose yield in continuous hydrolysis.

Table 1. Summary of the experimental parameters and results

| Experiment | Exp 1 (control) | Exp 2 | Exp 3 | Exp 4 | Exp 5 |
|---|---|---|---|---|---|
| Scheme | | | | | |
| Main features | - single streams, S:L=1:1<br><br>- in.the reactor: 20%DM, pH=5, 50°C<br><br>- enzyme 45mg/gr | - single streams, S:L=1:1<br><br>- in the reactor: 20%DM, pH=5, 50°C,<br><br>- enzyme 45 mg/gr -τ=10h | - Liquid from CSTR @10h,<br>- fresh solids<br>-fresh enzyme 45 mg/gr | Washed solids from batch and CSTR @10h<br><br>-water added, 8%DM<br><br>-no fresh enzyme added | - slurry S:L=1:1, 20%DM pH=5, 50°C , 1h<br><br>- Enzyme adsorption procedure 1, 1h, 45mg/gr -τ=10h |
| Composition @ enzyme addition (g/Kg) | | | | | |
| Glucose | 0.20 | 16.03 | 15.63 | NA | 0.84 |
| Xylose | 3.15 | 17.90 | 16.73 | NA | 2.68 |
| Cellobiose | <2 | <2 | <2 | NA | <2 |
| Xylobiose | 2.44 | 5.69 | 4.34 | NA | 0.95 |
| Glucoligomers | 3.87 | 5.38 | 4.26 | NA | 4.47 |
| Xyloligomers | 18.43 | 11.22 | 11.92 | NA | 16.21 |
| Insoluble glucans | 83.94 | 66.47 | 18.74 | NA | 63.91 |
| Insoluble xylans | 13.99 | 17.03 | 7.92 | NA | 17.22 |
| Other solubles | 37.72 | 23.41 | 21.99 | NA | 25.26 |
| Other insolubles | 56.12 | 60.71 | 18.51 | NA | 68.46 |
| Monomers | 3.35 | 33.93 | 32.36 | NA | 3.52 |
| Dimers | 2.44 | 5.69 | 6.25 | NA | 0.95 |
| Oligomers | 22.30 | 16.60 | 16.18 | NA | 20.68 |
| Insoluble sugars | 97.93 | 83.50 | 26.66 | NA | 81.13 |
| Soluble sugars | 28.08 | 56.22 | 54.79 | NA | 25.15 |

(continued)

| Experiment | Exp 1 (control) | Exp 2 | Exp 3 | Exp 4 | Exp 5 |
|---|---|---|---|---|---|
| Soluble sugars/ Insoluble sugars | 0.29 | 0.67 | 2.05 | NA | 0.31 |
| $U_{ads}/U_{in}$ (xylanase) | 63% | 11% | 5% | 5% (CSTR) 31% (batch) | 33% |
| $U_{ads}/U_{in}$ (endocellulase) | 69% | 18% | 8% | 2% (CSTR) 14% (batch) | 48% |
| Final glucose yield | 50% | 38% | 20% | 32% (CSTR) 46% (batch) | 48% |

| Experiment | Exp 6 | Exp 7 | Exp 8 | Exp 9 | Exp 10 |
|---|---|---|---|---|---|
| Scheme | | | | | |
| Main features | -As Exp 5 - Enzyme adsorption procedure 1, 40 min | - As Exp 5 - Enzyme adsorption procedure 1, 20 min | - As Exp 1 - DM 14% | -As Exp 6 - DM 14% | - As exp 5 - Enzyme adsorption procedure 2, 40 min |
| Composition @ enzyme addition (g/Kg) | As exp 5 | As exp 5 | | | As exp 5 |
| Glucose | 0.84 | 0.84 | 0.52 | 0.52 | 0.84 |
| Xylose | 2.68 | 2.68 | 1.91 | 1.91 | 2.68 |
| Cellobiose | <2 | <2 | <2 | <2 | <2 |
| Xylobiose | 0.95 | 0.95 | 0.87 | 0.87 | 0.95 |
| Glucoligomers | 4.47 | 4.47 | 2.74 | 2.74 | 4.47 |
| Xyloligomers | 16.21 | 16.21 | 12.46 | 12.46 | 16.21 |
| Insoluble glucans | 63.91 | 63.91 | 55.63 | 55.63 | 63.91 |
| Insoluble xylans | 17.22 | 17.22 | 7.74 | 7.74 | 17.22 |

(continued)

| Experiment | Exp 6 | Exp 7 | Exp 8 | Exp 9 | Exp 10 |
|---|---|---|---|---|---|
| Other solubles | 25.26 | 25.26 | 18.31 | 18.31 | 25.26 |
| Other insolubles | 68.46 | 68.46 | 39.82 | 39.82 | 68.46 |
| Monomers | 3.52 | 3.52 | 2.43 | 2.43 | 3.52 |
| Dimers | 0.95 | 0.95 | 0.87 | 0.87 | 0.95 |
| Oligomers | 20.68 | 20.68 | 15.19 | 15.19 | 20.68 |
| Insoluble sugars | 81.13 | 81.13 | 63.37 | 63.37 | 81.13 |
| Soluble sugars | 25.15 | 25.15 | 18.50 | 18.50 | 25.15 |
| Soluble sugars/ insoluble sugars | 0.31 | 0.31 | 0.29 | 0.29 | 0.31 |
| $U_{ads}/U_{in}$ (xylanase) | 35% | 38% | 60% | 29% | 50% |
| $U_{ads}/U_{in}$ (endocellulase) | 40% | 45% | 76% | 77% | 67% |
| Final glucose yield | 40% | 38% | 57% | 53% | 47% |

**Claims**

1. A process for producing a hydrolyzed mixture comprising glucose from a pre-treated ligno-cellulosic slurry comprising a slurry liquid and slurry solids comprised of lignin and water insoluble sugars comprising glucans, wherein the process comprises the steps of:

   a. Adsorbing at least a portion of a cellulase from an enzyme cocktail having an initial cellulase activity onto the slurry solids of the pre-treated ligno-cellulosic biomass slurry to create an adsorbed cellulase wherein the adsorbed cellulase has an adsorbed cellulase activity which is greater than 45% of the initial cellulase activity;
   b. Subjecting pre-treated ligno-cellulosic slurry to enzymatic hydrolysis to produce the hydrolyzed mixture.

2. The process of claim 1, wherein the pre-treated ligno-cellulosic slurry liquid comprises water soluble sugars.

3. The process of claim 2, wherein the concentration of the water soluble sugars in the pre-treated ligno-cellulosic slurry is greater than zero and less than a value selected from the group consisting of 55g/Kg, 50g/Kg, 40Kg/Kg, and 30g/Kg of the pre-treated ligno-cellulosic slurry.

4. The process of any of claims 2 to 3, wherein the concentration of the water soluble sugars in the pre-treated ligno-cellulosic slurry divided by the concentration of the water insoluble sugars in the pre-treated ligno-cellulosic slurry is value in the range of 0.1 to 0.65, 0.1 to 0.5, 0.1 to 0.4, 0.1 to 0.3, 0.1 to 0.2, 0.2 to 0.65, 0.3 to 0.65, 0.4 to 0.65, and 0.5 to 0.65.

5. The process of any of claims 2 to 4, wherein the pre-treated ligno-cellulosic slurry further comprises a concentration of water soluble sugar oligomers and the concentration of water soluble oligomers in the pre-treated ligno-cellulosic slurry is greater than zero and less than a value selected from the group consisting of 15g/Kg, 10g/Kg, 7.5g/Kg, 2.5g/Kg and 5g/Kg of the pre-treated ligno-cellulosic slurry.

6. The process of claim 5, wherein the water soluble sugar oligomers comprise xylooligomers, and the percent amount of xylooligomers by weight is at least a value selected from the group consisting of 70, 75, 80, 85 and 90 percent of the total amount of the water soluble oligomers in the pre-treated ligno-cellulosic slurry.

7. The process of any of claims 2 to 6, wherein the pre-treated ligno-cellulosic slurry further comprises a glucose concentration, and the concentration of glucose in the pre-treated ligno-cellulosic slurry is less than a value selected from the group consisting of 15 g/Kg, 10 g/Kg and 5 g/Kg of the pre-treated ligno-cellulosic slurry.

8. The process of any of claims 2 to 7, wherein the pre-treated ligno-cellulosic slurry further comprises a concentration of xylose, and the concentration of xylose in the pre-treated ligno-cellulosic slurry is less than a value selected from the group consisting of 15 g/Kg, 10 g/Kg and 5 g/Kg of the pre-treated ligno-cellulosic slurry.

9. The process of any of claims 2 to 8, wherein the pre-treated ligno-cellulosic slurry further comprises a concentration of cellobiose, and the concentration of cellobiose in the pre-treated ligno-cellulosic slurry is less than a value selected from the group consisting of 2 g/Kg, 1.5 g/Kg, 1 g/Kg, and 0.5 g/Kg of the pre-treated ligno-cellulosic slurry.

10. The process of any of claims 2 to 9, wherein the pre-treated ligno-cellulosic slurry further comprises a concentration of xylobiose, and the concentration of xylobiose in the pre-treated ligno-cellulosic slurry is less than a value selected from the group consisting of 5.5 g/Kg 5 g/Kg, 4 g/Kg, 3 g/Kg, 2 g/Kg, and 1g/Kg of the pre-treated ligno-cellulosic slurry.

11. The process of any of claims 1 to 10, wherein the dry matter content of the pre-treated ligno-cellulosic slurry by weight is greater than a value selected from the group consisting of 10%, 15%, 18%, 20%, 22% and 25%.

12. The process of any of claims 1 to 11, wherein the enzyme cocktail further comprises proteins and the total amount of proteins of the enzyme cocktail per gr of glucans in the pre-treated ligno-cellulosic slurry is less than a value selected from the group consisting 10, 7.5, 5, 4, 3, and 2 mg.

13. The process of any of claims 1 to 12, wherein the adsorbed cellulase activity is at least a value selected from the group consisting of 50%, 55%, 60%, 65% 70%, 75%, 80% and 85% of the initial cellulase activity.

14. The process of any of claims 1 to 13, wherein the cellulase is selected from the group consisting of endoglucanase, cellobiohydrolase, beta-glucosidase, and combinations thereof.

15. The process of any of claims 1 to 14, wherein the enzyme cocktail further comprises a xylanase having an initial xylanase activity, and at least a portion of the xylanase is adsorbed on the solid pre-treated ligno-cellulosic feedstock, so that the adsorbed xylanase has a xylanase activity which is at least a value selected from the group consisting of 40%, 50%, 60%, 70% and 80% of the initial xylanase activity.

16. The process of any of claims 1 to 15, wherein the slurry solids of the pre-treated ligno-cellulosic slurry have been homogeneously dispersed in the pre-treated ligno-cellulosic slurry by applying a first shear to the pre-treated ligno-cellulosic slurry.

17. The process of any of claims 16, further comprising adjusting the pH of the pre-treated ligno-cellulosic slurry to a value in the range between 4.5 and 5.5, wherein the pH adjusting is done prior to or during applying the first shear to the pre-treated ligno-cellulosic slurry to obtain a homogeneous pH in the pre-treated ligno-cellulosic slurry.

18. The process of any of claims 1 to 17, wherein a second shear is applied to the pre-treated ligno-cellulosic slurry for a shearing time after the enzyme cocktail is introduced into the pre-treated ligno-cellulosic slurry or while the enzyme cocktail is being introduced into the pre-treated ligno-cellulosic slurry.

19. The process of claim 17, wherein the adsorption step occurs during an adsorption time which is greater than 0 seconds and less than a value selected from the group consisting of 5 hours, 1 hour, 40 minutes, 20 minutes, 10 minutes, 5 minutes, and 1 minute.

20. The process of any of claim 18 to 19, wherein the first shear and the second shear are applied by means of a unitary shear device comprising more than one shear zone.

21. The process of claim 20, wherein the shear device is selected from the group consisting of an homogenizer, a compounder, an extruder, and a mixer.

22. The process of any of claims 1 to 21, wherein the enzymatic hydrolysis is conducted in a continuous manner.

23. The process of claim 22, wherein the enzymatic hydrolysis comprises the steps of:

a. introducing the pre-treated ligno-cellulosic slurry in a first vessel;
b. Maintaining the pre-treated ligno-cellulosic slurry at hydrolysis conditions to produce a partially hydrolyzed mixture comprising glucooligomers from the glucans of the pre-treated ligno-cellulosic feedstock;
c. removing the partially hydrolyzed mixture from the first vessel.

24. The process of claim 22, wherein the enzymatic hydrolysis further comprises the step of hydrolyzing the partially hydrolyzed mixture in at least a second vessel to produce the hydrolyzed mixture.

25. The process of claim 24, wherein the hydrolysis of the partially hydrolyzed mixture is conducted in a continuous manner.

26. The process of claim 24, wherein the hydrolysis of the partially hydrolyzed mixture is conducted in a batch mode.

Figure 1

Figure 2

Figure 3

Figure 4

Figure 5

Figure 6

Figure 7

Figure 8

Figure 9

Europäisches
Patentamt

European
Patent Office

Office européen
des brevets

# EUROPEAN SEARCH REPORT

Application Number

EP 15 42 5019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ROMANÍ ALOIA ET AL: "Integrated approach for effective bioethanol production using whole slurry from autohydrolyzed Eucalyptus globulus wood at high-solid loadings", FUEL, vol. 135, 2014, pages 482-491, XP029043257, ISSN: 0016-2361, DOI: 10.1016/J.FUEL.2014.06.061 * abstract * * page 483, right-hand column, paragraph 2 - page 486, right-hand column, last paragraph; tables 1,2 * * page 487, left-hand column, paragraph 1 - page 488, right-hand column, paragraph 1; figures 1,5 * * page 490, left-hand column, paragraph 2 * | 1-26 | INV. C12P19/14 C08H8/00 C08L97/02 C13K1/00 C13K13/00 |
| Y | ZHENG YINGFU ET AL: "Temperature sensitivity of cellulase adsorption on lignin and its impact on enzymatic hydrolysis of lignocellulosic biomass", JOURNAL OF BIOTECHNOLOGY, ELSEVIER SCIENCE PUBLISHERS, AMSTERDAM, NL, vol. 166, no. 3, 4 May 2013 (2013-05-04), pages 135-143, XP028573813, ISSN: 0168-1656, DOI: 10.1016/J.JBIOTEC.2013.04.018 * abstract * * page 137, left-hand column, paragraph 1 - page 142, right-hand column, paragraph 2 * | 1-26 | TECHNICAL FIELDS SEARCHED (IPC) C12P |

-----

-----

-/--

The present search report has been drawn up for all claims

| Place of search | Date of completion of the search | Examiner |
|---|---|---|
| Berlin | 19 June 2015 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 15 42 5019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BENNY PALMQVIST ET AL: "Effect of mixing on enzymatic hydrolysis of steam-pretreated spruce: a quantitative analysis of conversion and power consumption", BIOTECHNOLOGY FOR BIOFUELS, BIOMED CENTRAL LTD, GB, vol. 4, no. 1, 11 May 2011 (2011-05-11), page 10, XP021102043, ISSN: 1754-6834, DOI: 10.1186/1754-6834-4-10 * abstract * * page 2, right-hand column, paragraph 2 - page 3, left-hand column, paragraph 1 * * page 3, right-hand column, last paragraph - page 4, right-hand column, paragraph 2 * * page 5, right-hand column, last paragraph - page 7, left-hand column, paragraph 1 * * page 7, right-hand column, paragraph 3 * | 1-26 | |
| Y | WO 2010/011957 A2 (UNIV CALIFORNIA [US]; WYMAN CHARLES E [US]; KUMAR RAJEEV [US]) 28 January 2010 (2010-01-28) * page 2, paragraph [0007] * * page 6, paragraph [0037] - page 7, paragraph [0038] * * page 9, paragraph [0047] - page 11, paragraph [0052] * * page 15, paragraph [0061] * * example 1 * | 1-26 | |

-----

-----

-/--

TECHNICAL FIELDS
SEARCHED (IPC)

The present search report has been drawn up for all claims

| Place of search | Date of completion of the search | Examiner |
|---|---|---|
| Berlin | 19 June 2015 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                              

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YANPIN LU ET AL: "Cellulase Adsorption and an Evaluation of Enzyme Recycle During Hydrolysis of Steam-Exploded Softwood Residues", APPLIED BIOCHEMISTRY AND BIOTECHNOLOGY, HUMANA PRESS, INC, UNITED STATES, vol. 98/100, 1 January 2002 (2002-01-01), pages 641-654, XP007913098, ISSN: 0273-2289 * page 645, paragraph 3 - page 652, paragraph 1; figures 1-5 * | 1-26 | |
| Y | RAJEEV KUMAR ET AL: "Cellulase adsorption and relationship to features of corn stover solids produced by leading pretreatments", BIOTECHNOLOGY AND BIOENGINEERING 20090601 JOHN WILEY AND SONS INC. USA, vol. 103, no. 2, 1 June 2009 (2009-06-01), pages 252-267, XP055197101, DOI: 10.1002/BIT.22258 * page 256, left-hand column, paragraph 3 - page 261, left-hand column, paragraph 2; figures 1-4,6; tables IV, V * | 1-26 | |
| Y | AMADEUS PRIBOWO ET AL: "The adsorption and enzyme activity profiles of specific Trichoderma reesei cellulase/xylanase components when hydrolyzing steam pretreated corn stover", ENZYME AND MICROBIAL TECHNOLOGY, vol. 50, no. 3, 1 March 2012 (2012-03-01), pages 195-203, XP055196289, ISSN: 0141-0229, DOI: 10.1016/j.enzmictec.2011.12.004 * abstract * * page 202, left-hand column, paragraph 4 - right-hand column, paragraph 2 * | 1-26 | |

TECHNICAL FIELDS
SEARCHED        (IPC)

The present search report has been drawn up for all claims

| Place of search | Date of completion of the search | Examiner |
|---|---|---|
| Berlin | 19 June 2015 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 42 5019

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2015

| Patent document cited in search report | Publication date | Patent family member(s) | Publication date |
|---|---|---|---|
| WO 2010011957 A2 | 28-01-2010 | US 2011201084 A1<br>WO 2010011957 A2 | 18-08-2011<br>28-01-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012036768 A1
- US 8603789 B
- US 2011312033 A
- US 20130236933 A
- WO 9117243 A
- WO 9117244 A
- WO 9105039 A
- WO 9315186 A
- US 5275944 A
- WO 9602551 A
- US 5536655 A
- WO 0070031 A
- WO 05093050 A
- WO 2011059740 A
- WO 2009042871 A
- WO 2010141325 A
- WO 2006074435 A
- WO 2010057086 A
- WO 2005047499 A
- WO 2002095014 A
- WO 2007019442 A
- WO 2010088387 A
- WO 2011035029 A
- WO 2008057637 A
- WO 9813465 A
- WO 98015619 A
- WO 98015633 A
- WO 9906574 A
- WO 9910481 A
- WO 99025847 A
- WO 99031255 A
- WO 2002101078 A
- WO 2003027306 A
- WO 2003052054 A
- WO 2003052055 A
- WO 2003052056 A
- WO 2003052057 A
- WO 2003052118 A
- WO 2004016760 A
- WO 2004043980 A
- WO 2004048592 A
- WO 2005001065 A
- WO 2005028636 A
- WO 2005093050 A
- WO 2005093073 A
- WO 2006074005 A
- WO 2006117432 A
- WO 2007071818 A
- WO 2007071820 A
- WO 2008008070 A
- WO 2008008793 A
- US 5457046 A
- US 5648263 A
- US 5686593 A
- WO 9421785 A
- WO 2006078256 A
- WO 2011041405 A
- WO 2010126772 A
- WO 2009079210 A
- WO 2011057083 A
- WO 2010108918 A
- WO 2009073709 A
- WO 2005001036 A
- WO 2010014880 A
- WO 2009042846 A
- WO 2009076122 A
- WO 2009127729 A
- WO 2010053838 A
- WO 2010065448 A
- WO 2006114094 A
- WO 2009073383 A
- WO 2010014706 A
- WO 2009068565 A
- US 20110312051 A

### Non-patent literature cited in the description

- **ZHIZHUANG XIAO et al.** Effects of sugar inhibition on cellulases and β-glucosidase during enzymatic hydrolysis of softwood substrates. *Applied Biochemistry and Biotechnology,* 2004, vol. 113-116, 1115

- **YOUNGMI KIM et al.** Soluble inhibitors/deactivators of cellulase enzymes from lignocellulosic biomass. *Enzyme and Microbial Technology,* 2011, vol. 48, 408-415

- **PAVLE ANDRIĆ et al.** Reactor design for minimizing product inhibition during enzymatic lignocellulose hydrolysis: I. Significance and mechanism of cellobiose and glucose inhibition on cellulolytic enzymes. *Biotechnology Advances,* 2010, vol. 28, 308-324
- **D. HUMBIRD et al.** Process Design and Economics for Biochemical Conversion of Lignocellulosic Biomass to Ethanol Dilute-Acid Pretreatment and Enzymatic Hydrolysis of Corn Stover. *Technical Report NREL/TP-5100-47764,* May 2011
- **TEERI.** Crystalline cellulose degradation: New insight into the function of cellobiohydrolases. *Trends in Biotechnology,* 1997, vol. 15, 160-167
- **TEERI et al.** Trichoderma reesei cellobiohydrolases: why so efficient on crystalline cellulose?. *Biochem. Soc. Trans.,* 1998, vol. 26, 173-178
- **SHALLOM, D. ; SHOHAM, Y.** Microbial hemicellulases. *Current Opinion In Microbiology,* 2003, vol. 6 (3), 219-228
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase-Novel carbohydrate esterase produced by Schizophyllum commune. *FEBS Letters,* 2006, vol. 580 (19), 4597-4601
- **HERRMANN ; VRSANSKA ; JURICKOVA ; HIRSCH ; BIELY ; KUBICEK.** The beta-D-xylosidase of Trichoderma reesei is a multifunctional beta-D-xylan xylohydrolase. *Biochemical Journal,* 1997, vol. 321, 375-381
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **OOI et al.** *Nucleic Acids Research,* 1990, vol. 18, 5884
- **SAKAMOTO et al.** *Current Genetics,* 1995, vol. 27, 435-439
- **SAARILAHTI et al.** *Gene,* 1990, vol. 90, 9-14
- **KAWAGUCHI et al.** *Gene,* 1996, vol. 173, 287-288
- **DAN et al.** *J. Biol. Chem.,* 2000, vol. 275, 4973-4980
- **HENRISSAT B.** A classification of glycosyl hydrolases based on amino-acid sequence similarities. *Biochem. J.,* 1991, vol. 280, 309-316
- **HENRISSAT B. ; BAIROCH A.** Updating the sequence-based classification of glycosyl hydrolases. *Biochem. J.,* 1996, vol. 316, 695-696
- More Gene Manipulations in Fungi. Academic Press, 1991
- **BAILEY, J. E.** Biochemical Engineering Fundamentals. McGraw-Hill Book Company, 1986
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics, in Advances. **MOSIER et al.** Biochemical Engineering/Biotechnology. Springer-Verlag, 1999, vol. 65, 23-40